# EUROPÄISCHE PATENTSCHRIFT

(11) **EP 1 721 974 B1**
(45) Veröffentlichungstag und Bekanntmachung des Hinweises auf die Patenterteilung: **15.10.2008**
(21) Anmeldenummer: 05009771.6
(22) Anmeldetag: 04.05.2005
(51) Int. Cl.: C12N 15/10

(54) **Kompetitives n-Hybrid System**
Competitive n-hybrid system
Système concurrentiel d'n-hybride

(43) Veröffentlichungstag der Anmeldung: 15.11.2006
(73) Patentinhaber: Signalomics GmbH, 1090 Wien (AT)
(72) Erfinder: Arntz, Claudia, Dr., 49525 Lengerich (DE); Meinders, Danielle, 48565 Steinfurt (DE); Block, Christoph, Dr., 48151 Münster (DE)
(74) Vertreter: Von Renesse, Dorothea

(56) Entgegenhaltungen:
- US-A1- 2004 180 325
- FRIESE ANKE ET AL: "Synthesis and biological evaluation of cycloalkylidene carboxylic acids as novel effectors of Ras/Raf interaction" JOURNAL OF MEDICINAL CHEMISTRY, Bd. 45, Nr. 7, 28. März 2002 (2002-03-28), Seiten 1535-1542, XP002350269 ISSN: 0022-2623
- ESTOJAK JOANNE ET AL: "Correlation of two-hybrid affinity data with in vitro measurements" MOLECULAR AND CELLULAR BIOLOGY, Bd. 15, Nr. 10, 1995, Seiten 5820-5829, XP002350270 ISSN: 0270-7306
- JAITNER BIRGIT K ET AL: "Discrimination of amino acids mediating ras binding from noninteracting residues affecting Raf activation by double mutant analysis" JOURNAL OF BIOLOGICAL CHEMISTRY, Bd. 272, Nr. 47, 21. November 1997 (1997-11-21), Seiten 29927-29933, XP002350271 ISSN: 0021-9258

### SEQUENCE LISTING

<110> Signalomics
<120> "Kompetitives n-Hybrid-System"
<130> 45 271 K
<140> EP05009771
   <141> 2005-05-04
<160> 33
<170> PatentIn version 3.3
<210> 1
   <211> 1286
   <212> DNA
   <213> Saccharomyces cerevisiae
<400> 1
   gcaaagggaa gggatgctaa ggtagagggt gaacgttaca gaaaagcagg ctgggaagca      60
   tatttgagaa gatgcggcca gcaaaactaa aaaactgtat tataagtaaa tgcatgtata     120
   ctaaactcac aaattagagc ttcaatttaa ttatatcagt tattacccgg gatccgagct     180
   tggaattcga caggttatca gcaacaacac agtcatatcc attctcaatt agctctacca     240
   cagtgtgtga accaatgtat ccagcaccac ctgtaaccaa aacaatttta gaagtacttt     300
   cactttgtaa ctgagctgtc atttatattg aattttcaaa aattcttact ttttttttgg     360
   atggacgcaa agaagtttaa taatcatatt acatggcatt accaccatat acatatccat     420
   atctaatctt acttatatgt tgtggaaatg taaagagccc cattatctta gcctaaaaaa     480
   accttctctt tggaactttc agtaatacgc ttaactgctc attgctatat tgaagtacgg     540
   attagaagcc gccgagcggg tgacagccct ccgaaggaag actctcctcc gtgcgtcctc     600
   gtcttcaccg gtcgcgttcc tgaaacgcag atgtgcctcg cgccgcactg ctccgaacaa     660
   taaagattct acaatactag cttttatggt tatgaagagg aaaaattggc agtaacctgg     720
   ccccacaaac cttcaaatga acgaatcaaa ttaacaacca taggatgata atgcgattag     780
   ttttttagcc ttatttctgg ggtaattaat cagcgaagcg atgatttttg atctattaac      840
   agatatataa atgcaaaaac tgcataacca ctttaactaa tactttcaac attttcggtt     900
   tgtattactt cttattcaaa tgtaataaaa gtatcaacaa aaaattgtta atatacctct     960
   atactttaac gtcaaggaga aaaaactata atgactaaat ctcattcaga agaagtgatt    1020
   gtacctgagt tcaattctag cgcaaaggaa ttaccaagac cattggccga aaagtgcgga    1080
   attccaagct tggccaagcc cggatccgga gcttggctgt tgcccgtctc actggtgaaa    1140
   agaaaaacca ccctggcgcc caatacgcaa accgcctctc cccgcgcgtt ggccgattca    1200
   ttaatgcagc tggcacgaca ggtttcccga cttaatcgcc ttgcagcaca tccccctttc    1260
   gccagctggc gtaatagcga agaggc                                         1286
<210> 2
   <211> 31
   <212> DNA
   <213> artificial
<220>
   <223> Primer
<400> 2
   acgggtaccg caaagggaag ggatgctaag g 31
<210> 3
   <211> 30
   <212> DNA
   <213> artificial
<220>
   <223> Primer
<400> 3
   atcggtacct gaacgttaca gaaaagcagg 30
<210> 4
   <211> 31
   <212> DNA
   <213> artificial
<220>
   <223> Primer
<400> 4
   actactagtg cctcttcgct attacgccag c 31
<210> 5
   <211> 27
   <212> DNA
   <213> artificial
<220>
   <223> Primer
<400> 5
   agaactagtg gaagatcgca ctccagc 27
<210> 6
   <211> 31
   <212> DNA
   <213> artificial
<220>
   <223> Primer
<400> 6
   acaactagta acttttcggc caatggtctt g 31
<210> 7
   <211> 37
   <212> DNA
   <213> artificial
<220>
   <223> Primer
<400> 7
   actactagtc ctatagtttt ttctccttga cgttaaa 37
<210> 8
   <211> 681
   <212> DNA
   <213> artificial
<220>
   <223> RedStar (RFP)
<400> 8
   atgagtagat cttctaagaa cgtcatcaag gaattcatga gattcaaggt taaaatggaa 60
   ggtactgtta acggccacga attcgaaatc gaaggtgaag gtgagggtag accatatgaa 120
   ggtcacaaca cagtcaagtt gaaggttact aagggtggtc cactgccatt cgcttgggac 180
   atcttgtctc cacaattcca atacggttct aaggtctacg tcaagcaccc agctgacatt 240
   ccagactaca agaagttgtc cttcccagaa ggtttcaagt gggaaaggat catgaacttc 300
   gaagacggtg gcgttgttac tgttactcaa gactcctcct tgcaagacgg ttgtttcatc 360
   tacaaggtca agctcattgg tgtcaacttc ccatctgacg gtccagtcat gcaaaagaag 420
   actatgggtt gggaagcttc taccgaacgt ttgtacccaa gagacggtgt cttgaagggt 480
   gaaatccaca aggccttgaa gttgaaggac ggtggtcact acttggtcga attcaagtct 540
   atctacaagg ccaagaagca agtccaattg ccaggctatt actacgttga ctctaagttg 600
   gacatcatct ctcacaacga agactacact atcgtcgaac aatacgaacg tactgaaggt 660
   agacaccact tgttcttgta a 681
<210> 9
   <211> 34
   <212> DNA
   <213> artificial
<220>
   <223> Primer
<400> 9
   actactagtt atgagtagat cttctaagaa cgtc 34
<210> 10
   <211> 33
   <212> DNA
   <213> artificial
<220>
   <223> Primer
<400> 10
   tattccgcgg ttacaagaac aagtggtgtc tac 33
<210> 11
   <211> 781
   <212> DNA
   <213> Propionibacterium freudenreichii
<400> 11
   tcatgaccac tactttattg ccaggtactg ttactttggt tggtgcaggt ccaggtgatc 60
   cagaattggt tacagttgct ggtttaagag cagttcaaca agctgaagtt attctatacg     120
   atagattggc tccacaagat ttgttgtctg aagcatctga cgatgcagaa ttagttccag     180
   ttggtaaaat tccaagagga cattacgttc cacaggagga aattaatcaa ttattggttg     240
   ctcatgctag agaaggtaga aaagtagtta gattaaaggg tggagattct ttcgtttttg     300
   gtagaggtgg agaggaatgg caagcatgtg ctgaagctgg tattccagtt agagttattc     360
   caggagtttc ttctgctact gctggtccag ctttggctgg tattcctttg actcatagac     420
   atttggttca aggttttact gttgtttcag gtcatgtttc tccatcagat gaaagatcag     480
   aagttccttg gagacaattg gctaaggata gattgacttt ggttatttta atgggagttg     540
   ctcatatgag agatattgct ccagaattga tggctggagg tttaccagca gatactcctg     600
   ttagagtagt ttctaatgct tctttggctt ctcaggaatc ttggagaact actttgggag     660
   atgcagttgc tgatatggat gctcatcacg ttagaccacc tgctttggta gtagttggta     720
   ctttggctgg agtagatttg tctcatccag atcatagagc accatcagat cataactcga     780
   g                                                                     781
<210> 12
   <211> 1782
   <212> DNA
   <213> Saccharomyces cerevisiae
<400> 12
   atggtacgag acttagtgac attgccttca tcactgccct tgattactgc tggttttgct     60
   actgatcagg ttcatttgct tattggtaca gggtccacgg actcagtaag cgtttgtaag    120
   aatagaatcc actccatttt gaatgctggt ggtaatccca tagtagtgaa tccctcgtca    180
   ccaagccata ctaaacaatt acaattggaa tttggtaagt ttgcaaagtt cgaaatagta    240
   gaaagggagt ttaggttatc tgatttaact actttgggga gagttctggt atgcaaggta    300
   gtggatagag tattcgtaga tctacccata acacaaagtc gcctatgcga ggagatcttt    360
   tggcaatgcc aaaaactgag aattcccata aatacattcc acaaaccaga gttttctacc    420
   ttcaatatga ttcctacgtg ggtcgaccca aaaggaagtg gtttacaaat ctcagttact    480
   acgaatggga atggatacat cttggcaaac aggataaaaa gagatataat atcacactta    540
   cctccaaaca tatctgaggt ggtgataaac atggggtatt tgaaagaccg tattataaac    600
   gaagaccata aggccttgtt agaggaaaag tactaccaga ctgacatgtc attacctgga    660
   tttggctacg gcttagatga ggacggttgg gagagccata agtttaataa gctaattcgt    720
   gaatttgaaa tgaccagtag agaacagaga cttaagagaa ccagatggtt atctcagata    780
   atggagtatt acccgatgaa caagctgagt gacatcaagt tggaagattt cgagacttca    840
   tcttctccaa ataaaaagac aaagcaggaa actgtcacag agggtgtagt acctcctacc    900
   gatgaaaata ttgaaaacgg tacaaaacaa ctacaattat cggaagtgaa aaaagaggag    960
   ggacctaaaa aactagggaa gatttcttta gtcggaagtg gtccaggctc ggtatctatg   1020
   ctaacgatag gtgcattaca agaaataaag tctgcagata taatactggc agataaactg   1080
   gtaccgcaag ccattttaga tttaatacct ccaaaaactg aaaccttcat agccaaaaaa   1140
   tttcccggta atgcagaacg agcacaacag gaattactag ctaaaggttt agaatcgttg   1200
   gataatggat tgaaagtagt ccgtttgaag caaggtgatc cgtatatttt tggccgtggt   1260
   ggcgaggaat ttaatttctt caaagatcac ggatatattc ctgtggtttt accgggcata   1320
   agctcatccc tagcttgtac tgtattggct cagatacccg ctactcaacg tgatatagca   1380
   gaccaagtgc tcatatgtac tgggactggg agaaagggcg ctctgcctat aattcctgaa   1440
   tttgttgaaa gcagaaccac cgtctttcta atggcactgc atcgcgccaa cgttctgatc   1500
   acgggattat tgaagcatgg ctgggatggt gatgtccccg ctgcaattgt cgagagagga   1560
   tcgtgccctg accagcgtgt tactagaact cttcttaaat gggtaccaga agtcgtggag   1620
   gagattggtt caaggccccc cggtgtcttg gttgtaggca aggctgtgaa tgcattggtt   1680
   gaaaaagatc tgataaattt tgacgaatca agaaaatttg tcattgatga aggttttaga   1740
   gaatttgagg ttgatgtaga tagtctattt aagttatact aa                      1782
<210> 13
   <211> 32
   <212> DNA
   <213> artificial
<220>
   <223> Primer
<400> 13
   aattatccat ggtacgagac ttagtgacat tg 32
<210> 14
   <211> 45
   <212> DNA
   <213> artificial
<220>
   <223> Primer
<400> 14
   aattaactcg agttgtataa cttaaataga ctatctacat caacc 45
<210> 15
   <211> 36
   <212> DNA
   <213> artificial
<220>
   <223> Primer
<400> 15
   atccttgagc tctcagcagc tctgatgtag atacac 36
<210> 16
   <211> 43
   <212> DNA
   <213> artif-icial
<220>
   <223> Primer
<400> 16
   atcccccatg gctgataatg ggttagtagt ttataattat gtg 43
<210> 17
   <211> 488
   <212> DNA
   <213> saccharomyces cerevisiae
<400> 17
   tcagcagctc tgatgtagat acacgtatct cgacatgttt tatttttact atacatacat 60
   aaaagaaata aaaaatgata acgtgtatat tattattcat ataatcaatg agggtcattt 120
   tctgaaacgc aaaaaacggt aaatggaaaa aaaataaaga tagaaaaaga aaacaaacaa 180
   aggaaaggtt agcatattaa ataactgagc tgatacttca acagcatcgc tgaagagaac 240
   agtattgaaa ccgaaacatt ttctaaaggc aaacaaggta ctccatattt gctggacgtg 300
   ttctttctct cgtttcatat gcataattct gtcataagcc tgttcttttt cctggcttaa 360
   acatcccgtt ttgtaaaaga gaaatctatt ccacatattt cattcattcg gctaccatac 420
   taaggataaa ctaatcccgt tgttttttgg cctcgtcaca taattataaa ctactaaccc 480
   attatcag 488
<210> 18
   <211> 33
   <212> DNA
   <213> artificial
<220>
   <223> Primer
<400> 18
   atccccgcgg tagcttcaaa atgtttctac tcc 33
<210> 19
   <211> 38
   <212> DNA
   <213> artificial
<220>
   <223> Primer
<400> 19
   atcccccatg gtttgtaatt aaaacttaga ttagattg 38
<210> 20
   <211> 411
   <212> DNA
   <213> Saccharomyces cerevisiae
<400> 20
   tagcttcaaa atgtttctac tcctttttta ctcttccaga ttttctcgga ctccgcgcat 60
   cgccgtacca cttcaaaaca cccaagcaca gcatactaaa tttcccctct ttcttcctct 120
   agggtgtcgt taattacccg tactaaaggt ttggaaaaga aaaaagagac cgcctcgttt 180
   ctttttcttc gtcgaaaaag gcaataaaaa tttttatcac gtttcttttt cttgaaaatt 240
   tttttttttg atttttttct ctttcgatga cctcccattg atatttaagt taataaacgg 300
   tcttcaattt ctcaagtttc agtttcattt ttcttgttct attacaactt tttttacttc 360
   ttgctcatta gaaagaaagc atagcaatct aatctaagtt ttaattacaa a 411
<210> 21
   <211> 36
   <212> DNA
   <213> artificial
<220>
   <223> Primer
<400> 21
   atccccgcgg cagttcgagt ttatcattat caatac 36
<210> 22
   <211> 36
   <212> DNA
   <213> artificial
<220>
   <223> Primer
<400> 22
   atcccccatg gtttgtttgt ttatgtgtgt ttattc 36
<210> 23
   <211> 680
   <212> DNA
   <213> Saccharomyces cerevisiae
<400> 23
   cagttcgagt ttatcattat caatactgcc atttcaaaga atacgtaaat aattaatagt 60
   agtgattttc ctaactttat ttagtcaaaa aattagcctt ttaattctgc tgtaacccgt 120
   acatgcccaa aatagggggc gggttacaca gaatatataa catcgtaggt gtctgggtga 180
   acagtttatt cctggcatcc actaaatata atggagcccg ctttttaagc tggcatccag 240
   aaaaaaaaag aatcccagca ccaaaatatt gttttcttca ccaaccatca gttcataggt 300
   ccattctctt agcgcaacta cagagaacag gggcacaaac aggcaaaaaa cgggcacaac 360
   ctcaatggag tgatgcaacc tgcctggagt aaatgatgac acaaggcaat tgacccacgc 420
   atgtatctat ctcattttct tacaccttct attaccttct gctctctctg atttggaaaa 480
   agctgaaaaa aaaggttgaa accagttccc tgaaattatt cccctacttg actaataagt 540
   atataaagac ggtaggtatt gattgtaatt ctgtaaatct atttcttaaa cttcttaaat 600
   tctactttta tagttagtct tttttttagt tttaaaacac caagaactta gtttcgaata 660
   aacacacata aacaaacaaa 680
<210> 24
   <211> 45
   <212> DNA
   <213> artificial
<220>
   <223> Primer
   <400> 24
ctatataact ctgtagaaat aaagagtatc atctttcaaa gagct 45
<210> 25
   <211> 45
   <212> DNA
   <213> artificial
<220>
   <223> Primer
<400> 25
   ctttgaaaga tgatactctt tatttctaca gagttatata gacgt 45
<210> 26
   <211> 32
   <212> DNA
   <213> artificial
<220>
   <223> Primer
<400> 26
   aattccacat gtccgacccg agtaagacaa gc 32
<210> 27
   <211> 40
   <212> DNA
   <213> artificial
<220>
   <223> Primer
<400> 27
   attgccgcgg ttagtcgaca tctagaaaat ctacttgaag 40
<210> 28
   <211> 50
   <212> DNA
   <213> artificial
<220>
   <223> Primer
<400> 28
   tgaatactca tactcttcct gtttaaacat tattgaagca tttatcaggg 50
<210> 29
   <211> 52
   <212> DNA
   <213> artificial
<220>
   <223> Primer
<400> 29
   ttaaatcaat ctaaagtata tatgtttaaa cttggtctga cagttaccaa tg 52
<210> 30
   <211> 47
   <212> DNA
   <213> artificial
<220>
   <223> Primer
<400> 30
   aaaaaaccgt ttaaacagga agagtatgat tgaacaagat ggattgc 47
<210> 31
   <211> 50
   <212> DNA
   <213> artificial
<220>
   <223> Primer
<400> 31
   aaaaaaccgt ttaaacttgg tctgacagtc agaagaactc gtcaagaagg 50
<210> 32
   <211> 41
   <212> DNA
   <213> artificial
<220>
   <223> Primer
<220>
   <221> misc_feature
   <222> (14)..(15)
   <223> n is a, c, g, or t
<400> 32
   ctgccttatg aaannkctca aggtgagggg cctgcaacca g 41
<210> 33
   <211> 41
   <212> DNA
   <213> artificial
<220>
   <223> Primer
<220>
   <221> misc_feature
   <222> (15)..(16)
   <223> n is a, c, g, or t
<400> 33
   ccctcacctt gagmnntttc ataaggcagt catgcaagct c 41

## Beschreibung

Die Erfindung betrifft eine Weiterbildung des Zwei-Hybrid-Systems zur Identifikation hochaffiner Ligandeninteraktionen.

Das Zwei-Hybrid System wurde ursprünglich von Fields und Song 1989 entwickelt und wurde aufgrund der Möglichkeit, mit seiner Hilfe einen Interaktionspartner für ein bestimmtes Protein aus einer Genbank durch Screening in *S. cerevisiae* zu identifizieren, sehr populär und in breitem Ausmaß eingesetzt. Es basiert in seinen Grundzügen darauf, die transaktivierende Aktivität eines Transkriptionsfaktors, dessen Polypeptidkette zunächst in eine separate DNA-Bindungsdomäne und eine Transaktivierungsdomäne zerlegt wurde, jeweils an ein Protein zu fusionieren (Köderprotein bzw. Beuteprotein) und über die anschließende nicht-kovalente Interaktion dieser Fusionsproteine (Hybridproteine) wieder funktionell zu rekonstituieren.

Da in der ursprünglichen Konzeption des Zwei-Hybrid Systems (*Yeast Two Hybrid*) posttranslationale Modifikationen der interagierenden Proteine nicht berücksichtigt werden konnten, wurde es einige Jahre später auf das sog. Drei-Hybrid-System erweitert. Damit konnte z.B. durch Expression einer dritten Komponente wie z.B. einer Proteinkinase, die Protein-Protein Interaktion in Abhängigkeit von der Phosphorylierung durch eine Proteinkinase detektiert werden (Osborne et al, 1995). Ebenso wurde gezeigt, daß in einem Drei-Hybrid System die Anwesenheit eines für die Protein-Protein Interaktion essentiellen Partners nachgewiesen werden kann, der an der Bildung eines ternären Komplexes beteiligt ist (Zhang und Lautar, 1996).

In ihrer ursprünglichen Konfiguration wurde bei den Zwei- bzw. Drei-Hybrid Systemen durch die Rekonstitution des Transkriptionsfaktors über die erfolgreiche Interaktion der Fusionsproteine ein für das Wachstum der Hefen essentielles Gen exprimiert *(forward hybrid system).* Später wurde für den Nachweis der Bindung ein Reportergen (z.B. lacZ) eingesetzt, das durch eine Farbreaktion eines Substrats (z.B. X-Gal) mit präzipitierendem Produkt eine einfache Identifizierung von Reportergen exprimierenden Kolonien oder - bei Einsatz eines löslichen Substrats - auch eine Quantifizierung der lacz Aktivität ermöglichte (BioTechniques 2000, 29, 278-288, Jaitner et al., 1997).

Es wurde auch eine Umkehrung des ursprünglichen Screening-Ansatzes entwickelt (reverses n-Hybrid), bei dem ein toxisches Gen durch eine Protein-Protein Interaktion transkribiert wird und erst die Störung der Protein-Protein Interaktion das Wachstum der Hefezellen ermöglicht (Vidal et al., 1996). Eine Hemmung der Interaktion konnte aber auch im forward n-Hybrid durch die Expression eines Inhibitors erreicht werden (Tirode et al., 1997).

Die Nutzung des Hybrid Systems ist aber nicht auf drei Komponenten begrenzt. Grundsätzlich sind wohl alle Proteinkomplexe mit einer ausreichenden Interaktionsaffinität für das Hybrid System geeignet. So wurde die Verwendung eines Vier-Hybrid Systems bereits gezeigt (Sandrock und Egly, 2001). Der Einsatz von vier Komponenten in einem System findet auch im "Duat-Baif Zwei-Hybrid System statt. Hierbei werden jeweils zwei interagierende Zwei-Hybrid Paare mit unterschiedlichen Reportergenen eingesetzt, um eine schnellere Diskriminierung zwischen spezifischen und unspezifischen Interaktionen im Zwei-Hybrid Screening zu ermöglichen (Serebrüskü et al., JBC 1999). Dieser Ansatz vereinfacht die qualitative Bestimmung der Spezifität von im Zwei-Hybrid System gefundenen Interaktionspartnern.

Im klassischen Zwei-Hybrid System findet die Protein-Protein Interaktion innerhalb des Zellkerns statt. Dieser ist jedoch nicht für alle Protein-Protein Interaktionen ein geeignetes Zellkompartiment. Für Interaktionen, für die eine Lokalisation im Zellkern ungeeignet ist, wurden Systeme entwickelt, in denen die zu detektierenden Interaktionen z.B. entweder im Cytoplasma oder an der Zellmembran stattfinden (Aronheim et al., 1997).

Mit der gleichzeitigen Verwendung eines selektiven Wachstumsmarkers (z.B. His3) und einem enzymatischen Reportergens (z.B. lacZ) für die etablierten Farb- und Fluoreszenzsubstrate wurde bereits früh der Versuch unternommen, das Zwei-Hybrid System nicht nur zur Detektion von Protein-Protein-Interaktionen - also als qualitativen Ansatz - einzusetzen, sondern auch zur Quantifizierung von Interaktionen zu nutzen. Eine erste vergleichende Studie unter Verwendung verschiedener Transkriptionsfaktoren konnte jedoch lediglich eine Eignung des Zwei-Hybrid Systems für semiquantitative Studien zeigen (Estojak et al., 1995). Eine Studie unter Verwendung von Punktmutanten zeigte eine quantitative Korrelation im Zwei-Hybrid System zwischen *in vivo* und *in vitro* Daten im nano- bis mikromolaren Affinitätsbereich (Jaitner et al., 1997).

Die in diesen Studien adressierte Problematik der quantitativen Bestimmung von Protein-Protein Interaktionen im Zwei-Hybrid System wurde in einer jüngeren Studie wieder aufgegriffen, in der der möglichst breite Einsatz von n-Hybrid Systemen beim Interaktions-Screening im Bereich der Medikamentenentwicklung untersucht wurde (de Felipe et al., 2004).

Für den Bereich der Medikamentenentwicklung und der Entwicklung von Diagnostika ist es von besonderer Bedeutung, die möglichen Interaktionen zwischen den beteiligten Bindungspartnern sowohl über einen sehr weiten Affinitätsbereich ("breiter dynamischer Bereich") quantifizieren zu können als auch gleichzeitig die Spezifität der Bindungen nachzuweisen, da pharmazeutische Wirkstoffe oder pharmazeutisch nutzbare Proteine eine sehr hohe Affinität und eine sehr hohe Spezifität aufweisen sollten.

Die aktuellen Arbeiten von de Felipe et al. (2004) machen in dieser Hinsicht jedoch die Grenzen der bekannten Hybrid-Systeme deutlich. Es wurde nämlich erkannt, daß der für die Affinitätsuntersuchungen zur Verfügung stehende dynamische Bereich lediglich ein bis zwei Größenordnungen umspannt. Um den gesamten erforderlichen Bereich abdecken zu können, müsste daher eine Mehrzahl unterschiedlicher Systeme verwendet werden.

Eigene Arbeiten bestätigen diese Aussagen. Diese Arbeiten basieren auf Untersuchungen zur Korrelation zwischen der biochemischen Affinität eines Beuteproteins zu dem Köderprotein und dem quantitativen Ergebnis der Interaktionsanalyse über den Nachweis an exprimiertern Reportergen (Readout) im Zwei-Hybrid System auf. Bei der untersuchten Interaktion handelt es sich um die Bindung zwischen Ras und der Ras-bindenden Domäne (RafRBD) der Proteinkinase Raf. Während Mutanten der RafRBD, die eine *verschlechterte* Affinität zu Ras aufweisen, im Zwei-Hybrid System eindeutig von der Wildtyp RafRBD (RafRBD-wt) unterschieden werden konnten (Jaitner et al., 1997), scheiterten alle Versuche, eine RafRBD-Mutante, die mit einer *erhöhten* Affinität zu Ras in der Literatur beschrieben ist (RafRBD-A85K, Burgess et al., 2000) - wie auch in eigenen biochemischen Messungen bestätigt wurde - durch eine erhöhte Reportergenaktivität im Zwei-Hybrid System zu identifizieren. Der Einsatz der RafRBD Mutante RafRBD-A85K mit nach biochemischen Messungen erhöhter Affinität zu Ras konnte demnach bislang im Zwei-Hybrid System nicht von der Wildtypform RafRBD-wt unterschieden werden. Dies lässt den Schluß zu, daß diese Mutante außerhalb des dynamischen Bereichs des Zwei-Hybrid Systems liegt.

Um gleichwohl einen hochaffinen Bindungspartner identifizieren zu können, wäre es denkbar, verschiedene unterschiedliche n-Hybrid Systeme zu etablieren, um damit in der Zusammenschau ein Screening über den gewünschten weiten dynamischen Bereich zu erreichen, und insbesondere auch zu erlauben, Proteine mit therapeutisch und diagnostisch relevanten hohen Affinitäten zu identifizieren. Dies ist jedoch in der Praxis kaum durchführbar. Insbesondere kann damit nicht gewährleistet werden, daß Proteine mit entsprechend verbesserten Eigenschaften in den Screenings sicher identifiziert werden können, da die Grenzen des dynamischen Bereichs im Einzelnen nicht vorhergesagt werden können.

US 2004/180325 (Edwards et. al.) zeigt ein Hybrid-System mit einstellbarer Sensitivität. Die Einstellung erfolgt auf dem Niveau der Promotoren der beiden chimären Proteine (induzierbare Promotoren, die durch die Konzentration einer Substanz regulierbar sind).

Die Aufgabe der vorliegenden Erfindung besteht daher darin, das bekannte Hybrid-System dahingehend weiterzuentwickeln, daß ein größerer dynamischer Bereich für die Affinitätsuntersuchungen zur Verfügung steht, insbesondere um damit hochaffine Liganden-Interaktionen selektieren zu können.

Diese Aufgabe wird gelöst durch ein Verfahren gemäß Anspruch 1. Vorteilhafte Weiterentwicklungen sind jeweils Gegenstand der Unteransprüche bzw. der unabhängigen Nebenansprüche.

Der Erfindung liegt der Gedanke zugrunde, das bekannte Hybrid-System so durchzuführen, daß die Bindungsreaktion zwischen dem ersten Liganden (erstes Hybrid- oder Fusionsprotein) und dem zweiten Liganden (zweites Hybrid- oder Fusionsprotein) durch die Wahl geeigneter Reaktionsbedingungen bewusst -verschlechtert" wird. Eine Verschlechterung im Sinne der vorliegenden Erfindung ist immer dann gegeben, wenn die Lage des Gleichgewichts (Fließgleichgewichts) der Reaktion der Bildung eines Ligandenkomplexes zu Gunsten der Liganden (Ausgangsprodukte) verschoben wird. Die Bindungsreaktion zwischen den Liganden ist daher gehemmt bzw. verlangsamt. Der Ligandenkompiex ist gegenüber den Liganden durch einen funktionalen Transkriptionsfaktor definiert.

Wird einer der Liganden des Ausgangssystems jedoch z.B. durch einen Liganden mit einer deutlich höheren Affinität zu dem jeweils anderen Bindungspartner ersetzt, wird die "Verschlechterung" des Systems überwunden und es bilden sich im Vergleich zu der Ausgangslage entsprechend mehr Ligandenkomplexe. Die Verwendung eines höher affinen Liganden führt daher gegenüber der gestörten Ausgangslage zu einer verstärkten Expression des Reportergens, dessen Aktivität quantitativ nachweisbar ist. Dies ermöglicht insbesondere die Darstellung der relativen Affinität eines Interaktionspaares gegenüber einem Vergleichspaar.

Durch die Veränderungen der Reaktionsbedingungen kann der dynamische Bereich, innerhalb dessen die Affinitätsuntersuchungen möglich sind, je nach Fragestellung und gewünschter Affinität des gesuchten Liganden angepasst oder gesteigert werden. So kann eine Ausgangsbank von Fusionsproteinen mehrere Zyklen des erfindungsgemäßen Verfahrens durchlaufen, wobei jeder Zyklus mit geänderten Bedingungen wiederholt werden kann. Damit stellt das erfindungsgemäße Verfahren ein System bereit, durch das z.B. Proteine mit hoher Affinität zu dem Köderprotein selektiert werden. In diesem repetitiven Einsatz kann das erfindungsgemäße Verfahren somit als Selektionsverfahren dienen und Liganden/Mutanten mit theoretisch unbegrenzt hoher Affinität darstellen.

Das erfindungsgemäße Verfahren eignet sich vor allem als Screening-Verfahren zum Vergleich affiner und hochaffiner Liganden. Um diesen Vergleich zu ermöglichen, kann z.B. die Reportergen-Expression eines Wildtyp-Liganden als Referenzwert (100%) angesetzt werden. Die Affinitäten von Mutanten dieses Wildtyps lassen sich davon ausgehend als relative Größen zu der Affinität des Wildtyps darstellen.

Erfindungsgemäß wird ein Kompetitor zur "Verschlechterung" der Bindungsreaktion eingesetzt. Der Kompetitor bindet an eines der Hybridproteine und hemmt oder verzögert damit im Sinne eines kompetitiven oder nicht-kompetitiven Hemmstoffes die Ausbildung des Ligandenkomplexes. Erfindungsgemäß ist es möglich, einen Kompetitor sowohl für das Beute- als auch für das Köderprotein einzusetzen. Sollte ein Kompetitor für das Beuteprotein gewählt werden - und somit die Ausbildung des Ligandenkomplexes dem Grunde nach verschlechtert werden - sollte aber die Affininität des Beuteproteins zu dem Köderprotein die Affinität des eingesetzten Kompetitors zu dem Köderprotein übersteigen, wird das Gleichgewicht der Ligandenbindungsreaktion wieder zu Gunsten der Ligandenkomplexbindung beeinflußt. Damit wird das hochaffine Beuteprotein durch die entsprechend hohe Reportergen-Expression quantitativ nachweisbar. Diese hochaffine Reaktion wäre nicht nachweisbar, wenn die Nachweisgrenze des Systems bereits überschritten wäre.

Die Feineinstellung des Systems kann in diesem Fall entscheidend über die Wahl des Kompetitors und seine Affinität zu dem Hybridprotein beeinflusst werden. Ebenso ist die Konzentration des Kompetitors von erheblicher Bedeutung, da die Konzentration eines Reaktanden bekanntlich die Gleichgewichtslage einer Reaktion maßgeblich bestimmt.

Ein besonderer Vorteil dieser Ausführungsform besteht darin, daß durch die Wahl eines *per se* spezifischen Kompetitors auch eine Spezifitätserhöhung des durch das repetitive Selektieren identifizierten Liganden (z.B. Beuteproteins) verbunden ist. Indem nämlich ein zu dem nativen Bindungspartner des Köderproteins ähnliches Protein - das demnach eine entsprechend hohe Spezifität aufweist - eingesetzt wird, bleiben im weiteren alle Beuteproteine unberücksichtigt, die eine geringere Spezifität besitzen. Beuteproteine mit einer "unspezifischen Bindung" werden demnach ausgeschlossen.

Der Kompetitor wird in der Wirtszelle selber exprimiert. Dies hat einerseits den Vorteil, daß der Kompetitor bereits in der Zelle vorliegt und eröffnet darüber hinaus die Möglichkeit, die Expression des Kompetitors - und damit im Ergebnis seine für die Lage des Gleichgewichts der Bindungsreaktion wesentliche Konzentration - durch die Wahl eines geeigneten Promotors zu variieren. Somit kann die Beeinflussung des erfindungsgemäßen Verfahrens sowohl über die Wahl des Kompetitors als durch die Regulation seiner Expression modifiziert werden.

In einer besonders vorteilhaften Ausführungsform werden nach der Expression des Kompetitors in einer Wirtszelle die Wachstumsbedingungen durch die Beeinflussung von Selektionsmarkern abhängig von der Medienzusammensetzung variiert, um Interaktionen mit modifizierter Affinität spezifisch zu begünstigen. So können beispielsweise die transformierten Wirtszellen auf einem Selektivmedium mit Aminotriazol als kompetitivem Hemmstoff für die His-Expression kultiviert werden. Bei gleicher Protein-Protein-Interaktion korrespondiert der Reportergen-Readout mit dem hohen Selektionsdruck auf dem His3-Gen. Durch die Zugabe unterschiedlicher Konzentrationen von Aminotriazol werden nur Wirtszellen mit ausreichend hochaffinen Fusionsprotein selektiert. Die Affinität der Bindungspartner muß nämlich so groß sein, daß trotz des Wachstums auf einem kompetitiven His-Expressionshemmstoff noch eine ausreichende Menge His bzw. das Reportergen exprimiert wird. Die Konzentration des Selektionsmarkers im Medium bestimmt - in Wechselwirkung mit den übrigen Faktoren des erfindungsgemäßen Systems (z.B. Stärke des die Kompetitor-Expressoin bestimmenden Promotors) - die gewünschte Affinität des über die Wirtszelle selektierten Fusionsproteins.

### Beispiele

### 1. Prinzip des erfindungsgemäßen Verfahrens mit Kompetitor

Zusätzlich zu den interagierenden Hybridproteinen (Fusionsproteinen) des bekannten Zwei-Hybrid Systems wird in S. *cerevisiae* eine dritte Komponente exprimiert. Dabei handelt es sich im Falle der Mutagenese des Köderproteins vorzugsweise um das freie Wildtyp-Köderprotein. Im Falle der Mutagenese des Beuteproteins zur Identifikation hochaffiner Beuteproteine wird vorzugsweise das Wildtyp Beuteprotein als Kompetitor exprimiert.

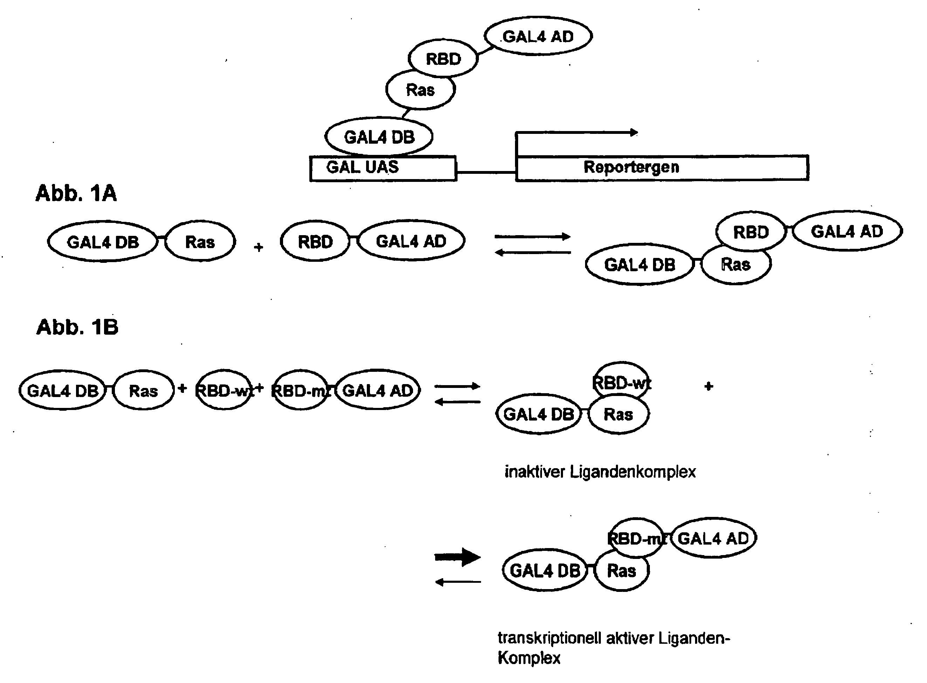

Das Grundprinzip des erfindungsgemäßen Verfahrens in dieser Ausführungsform ist in Abb. 1B für die Identifikation affiner RafRBD-Beuteproteinen unter Expression des Wildtyp-Beuteproteins als Kompetitor dargestellt. Bei der Erhöhung der Affinität des mutierten RafRBD-Fusionsproteins (RBD-mt) wird die Bildung des transkriptionell funktionalen Ligandenkomplexes gegenüber dem nicht-funktionalen (inaktiven) Komplex aus dem Kompetitor RafRBD-wt (RBD-wt) mit Ras bevorzugt. [Abkürzungen: RBD-mt: mutiertes RafRBD-Fusionsprotein; RafRBD-wt: Wildtyp-RafRBD; RBD: Ras-bindende Domäne; DB: DNA-Bindungsdomäne; AD: Transaktivierungsdomäne; UAS: Upstream-Aktivatorsequenz]

Die Abbildung 1A zeigt das bekannte Zwei-Hybrid System. In dem bekannten System wird nur die Wildtyp-Variante des Beuteproteins in dem Fusionsprotein eingesetzt. [Abkürzungen: RBD: Ras-bindende Domäne; DB: DNA-Bindungsdomäne; AD: Transaktivierungsdomäne; UAS: Upstream-Aktivatorsequenz]

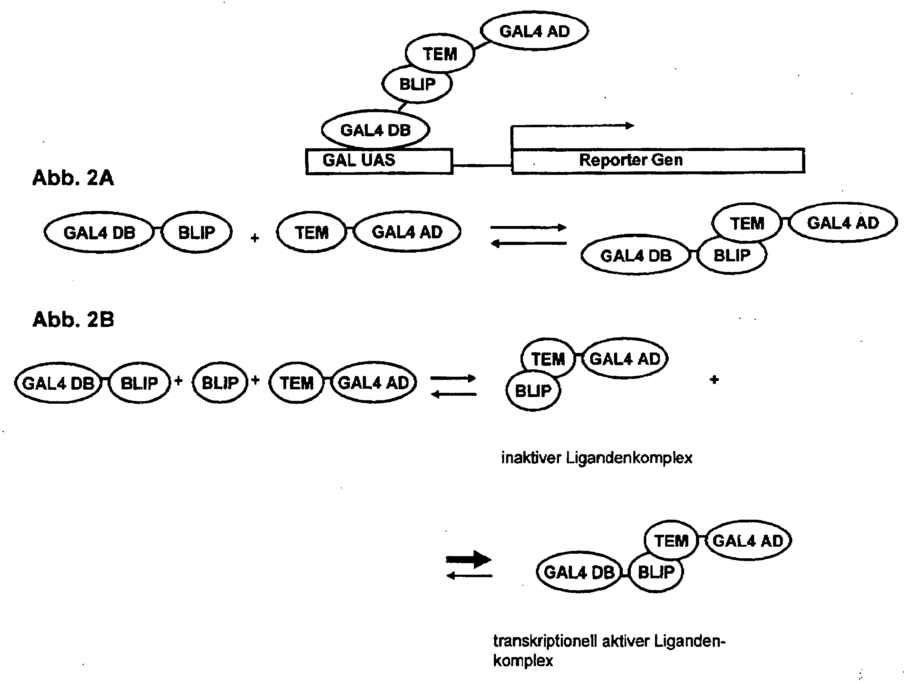

Das Verfahren nach Abb. 2B dient der Untersuchung der Interaktion zwischen den Proteinen BLIP als Köderprotein und TEM als Beuteprotein. Das Köderprotein wurde zuvor mutagenisiert (BLIP-mt) und als Fusionsprotein eingesetzt. In diesem System wird zusätzlich Wildtyp-BLIP (BLIP-wt) exprimiert und dient als Kompetitor. Bei Erhöhung der Affinität des mutierten BLIP-Fusionsproteins wird die Bildung des transkriptionell funktionalen (aktiven) Ligandenkomplex gegenüber dem

inaktiven Ligandenkomplex aus BLIP-wt und dem Fusionsprotein mit TEM bevorzugt. [Abkürzungen: DB: DNA-Bindungsdomäne; AD: Transaktivierungsdomäne; BLIP-wt: Wildtyp des Köderproteins; BLIP-mt: mutiertes Köderprotein; UAS: Upstream Aktivatorsequenz].

Im folgenden werden die Ergebnisse dargestellt, die mit Hilfe des erfindungsgemäßen Verfahrens unter Verwendung des Zwei-Hybridsystems aus Ras-Köderprotein und RafRDB-Beuteprotein mit zusätzlicher Expression des Wildtyp-Beuteproteins als Kompetitor erzielt wurden. Die Raf-RBD-Fusions-Beuteproteine wurden zuvor über eine dem Fachmann bekannte Mutagenese erzeugt. Grundsätzlich stehen für die Mutagenese alle üblichen Verfahren zur Verfügung.

Durch diese Ergebnisse wird deutlich, daß durch die Gegenwart des Kompetitors - in den dargestellten Beispielen RafRBD - der dynamische Bereich des erfindungsgemäßen Hybrid Systems jeweils so eingestellt werden kann, dass ein Protein mit verbesserten Eigenschaften zuverlässig identifiziert werden kann. Bevorzugte Variablen zur Einstellung des dynamischen Bereichs dieser Ausführungsform sind die Affinität des Kompetitors und die Expressionsstärke des Kompetitors (Stärke des Promotors). Durch einen wiederholten Einsatz dieses Prinzips in aufeinander folgenden Selektionsrunden (repetitive Selektion) mit jeweils in der Affinität bzw. Spezifität verbesserten Ausgangsproteinen (Beuteproteinen) und Kompetitoren lassen sich theoretisch unbegrenzt hoch affine und unbegrenzt hoch spezifische Proteine identifizieren.

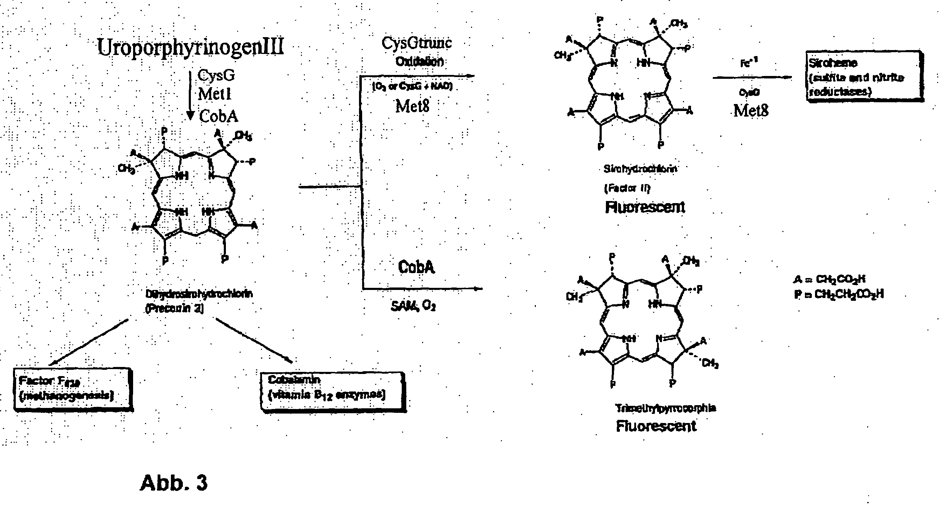

Das erfindungsgemäße Verfahren erlaubt es somit, Mutanten mit verbesserter Affinität zu generieren, die als hochaffine Proteine u.a. für diagnostische oder therapeutische Zwecke eingesetzt werden können. Diese Mutanten können durch ein direktes quantitatives Screening ermittelt werden. Als Reportergene für dieses quantitative Screening können beispielsweise Met1. CysG oder CobA eingesetzt werden (Roessner, C.A. 2002), die jeweils fluoreszente Derivate von Uroporphyrinogenlll liefern (Abb. 3; Ursprungsorganismen der Gene: *Propionibacterium freudenreichii:* CobA; *Saccharomyces cerevisiae:* Met1/Met8; *Escherichia coli:* CysG, nach Roessner 2002). Es ist auch der Einsatz von fluoreszenten Proteinen als Reportergen möglich, wie z.B. der Einsatz von Phycocyanin (Arntz et al., 2004) oder RedStar (Knop et al., 2002).

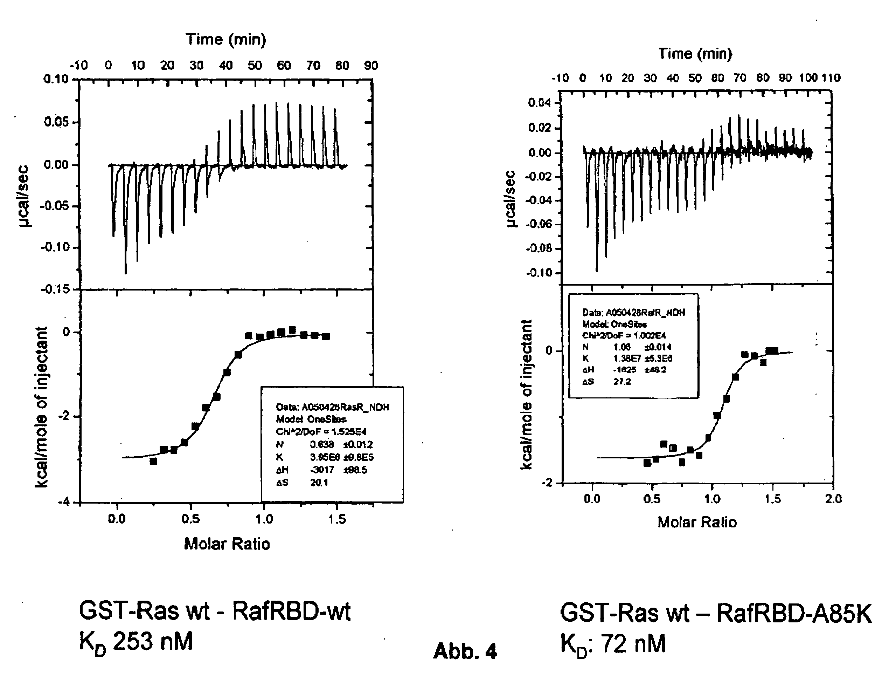

Zur Demonstration des Verfahrensprinzips für die Kombination der Mutagenese des Beuteproteins unter Expression des Wildtyp-Beuteproteins als Kompetitor wurde die RafRBD Mutante RafRBD-A85K eingesetzt, deren Bindungsaffinität bereits biochemisch charakterisiert ist. Die Dissoziationskonstante der RafRBD-A85K Mutante beträgt nach eigenen mikrokalorimetrischen Messungen in PBS-Puffer 72 nM im Vergleich zur Dissoziationskonstanten des entsprechenden RafRBD-wt Proteins von 253 nM (Abb. 4) (Amtz et al., unveröffentlicht).

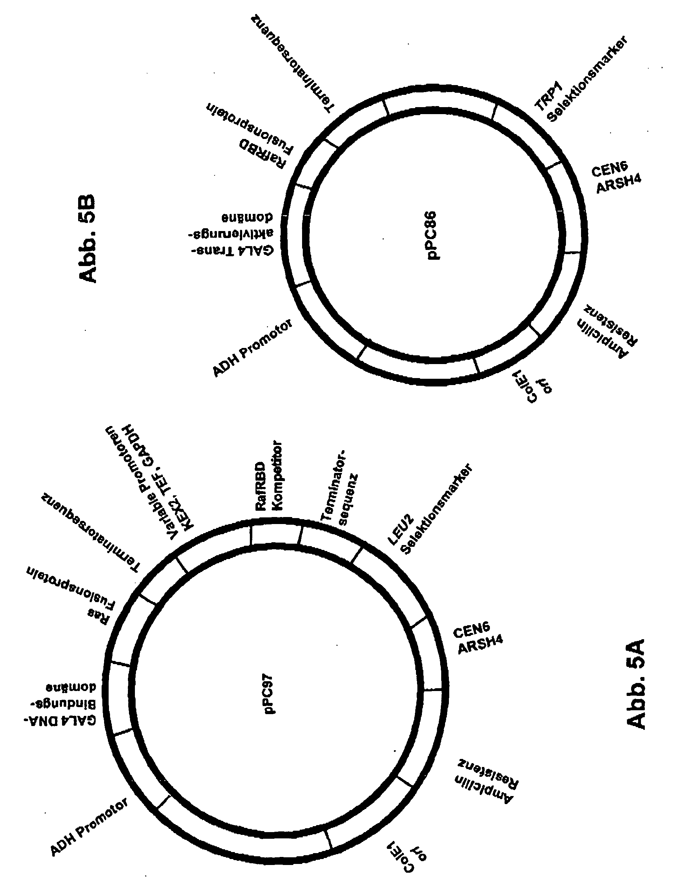

Zur Untersuchung der Ras/Raf-Interaktionen im erfindungsgemäßen Verfahren kann zur Bereitstellung des Köder-Fusionsproteines Ras-Ga14 ein aus dem Plasmid pPC97 abgeleitetes Plasmid verwendet werden. Auf diesem Plasmid wird auch der Kompetitor codiert. Einen Überblick über ein mögliches Plasmid gibt Abb. 5A. Die Abbildung 5B zeigt ein Plasmid auf Basis des pPC86, der das Beute-Fusionsprotein codiert.

Konstruktion von Hefestämmen für das erfindungsgemäße Verfahren

### a) Reportergene für das quantitative Screening

Für das erfindungsgemäße Verfahren wird ein Reportergen benötigt, das unter der Kontrolle eines regulierbaren Promotors steht und dessen Aktivität unmittelbar, qualitativ und quantitativ in der intakten Hefekolonie gemessen werden kann. Reportergene, die eine Fluoreszenz als Readout bewirken, erfüllen diese Ansprüche.

### (i) Wahl des Promotors für das Reportergen

In der Hefe Y190 wird die Expression des Reportergens ß-Galaktosidase durch die Stärke der Wechselwirkung von GAL4 Binde- und Aktivierungsdomäne reguliert, deren Gene durch zwei verschiedene Vektoren (pPC86 und pPC97, Chevray und Nathans, 1992) in die Hefezelle eingebracht werden.

Der Genotyp des Hefestamms Y190 ist als: "Mat a, leu2-3, 112, ura3-52, trp1-901, his3-Δ200, ade2-101, gal4Δgal80Δ, URA3::GAL-lacZ, LYS2::GAL-HIS3, cyh^{r}" bekannt. Dabei bedeutet URA3::GAL-lacZ, dass der fragliche Promotor (GAL; divergenter GAL1/GAL10-Promotor) in das URA3-Gen integriert worden ist.

Für das Ziel, ein Reportergen hinter diesen Promotor zu klonieren und in das Genom der Hefe zu integrieren, ist eine genaue Kenntnis der Situation an dieser Stelle im Genom unabdingbar (vgl. dazu Yocum et al. (1984) zur Integration des Ylp Plasmides pRY171 in das Genom von Y152 (entstanden aus YJO-Z, Leuther und Johnston. 1992), der der Vorläuferstamm von Y153 ist, aus dem wiederum Y190 entstanden ist).

Dann wurde die Entstehung des Plasmides pRY171, das den GAL-Promotor mit dem lacZ-Gen, beides hinter dem URA3-Gen, trägt, entschlüsselt, um Sequenzdaten zu erhalten: Yocum et al. (1984) haben dieses Plasmid aus dem Plasmid pLRIΔ3 durch die Entfernung der 2 µm Replikationsursprung-Sequenzen generiert. pLRIΔ3 entspricht dem Plasmid pRY131 bis auf einen Xhol Linker in der Mitte des divergenten Promotors. pRY131 wurde von West et al. (1984) generiert aus pLG 669 (Guarente und Ptashne, 1981) und pRY116. pLG 669 wiederum ist aus YEp24 entstanden, einem Plasmid mit pBR322-Rückgrat (Botstein et al., 1979).

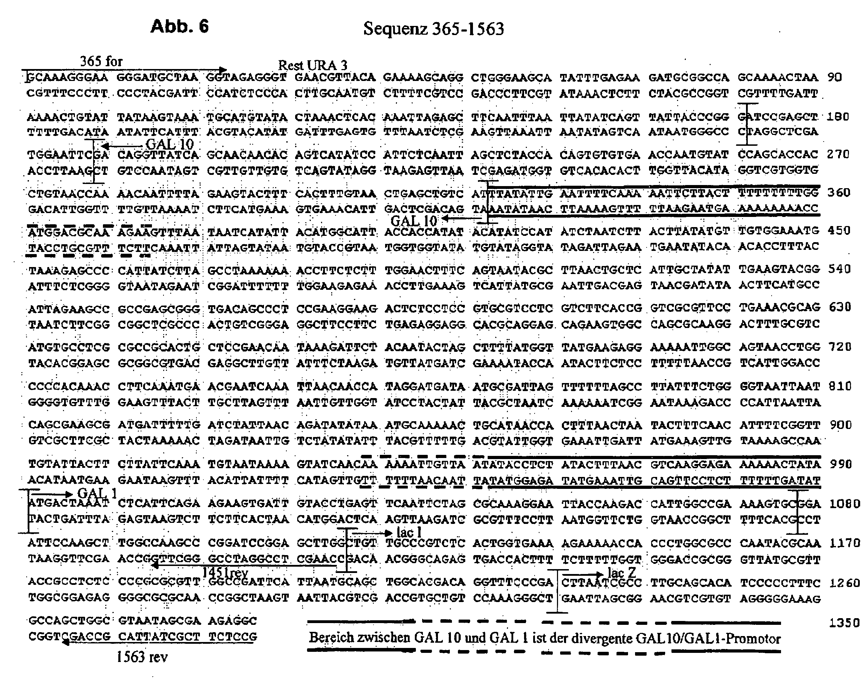

Aus diesen Daten wurde eine Sequenz generiert, nach der Primer (365-for, 394 for und 1563-rev sowie 1674-rev) synthetisiert wurden. Mit diesen wurde in einer PCR mit genomischer DNA aus Y190 dieses Stück DNA amplifiziert und sequenziert. Hierdurch wurde die tatsächliche Sequenz des divergenten GAL101GAL1-Promoters, hinter den das Reportergen zu klonieren war. Die Sequenz des PCR-Fragments 365-1563 ist beigefügt (Abb. 6, die wesentlichen Merkmale der Sequenz wurden eingezeichnet).

Mit diesen Primern wurden durch PCR die entsprechenden Stücke des Promotors amplifiziert und dann als Fusionsprodukt mit einem Fluorophor kloniert. Zusätzlich wurde noch der Promotor 365-1451 ausgewählt, der keinen lacl/5'lacZ-Anteil mehr besitzt. Dies geschah aus der Überlegung heraus, dass zusätzliche Genanteile die Expression des ausgewählten Fluorophors behindern könnten. Ein weiterer Promotor, bei dem auch der GALI-Anteil auf Null reduziert war (365-1366), wurde ebenfalls getestet. Bei dem Fluorophor handelte es sich um RedStar (Knop et al. 2002; s. Abschnitt (iii)).

Als bester Promotor stellte sich das Konstrukt 365-1451 (kein lacl/5'-lacZ mehr vorhanden) heraus; dieses wurde für alle folgenden Integrationen von Reportergenen in das Genom von *S. cerevisiae* eingesetzt.

### Verwendete Primer:

| | |
|---|---|
| 365-for | ACGGGTACCGCAAAGGGAAGGGATGCTAAGG (Kpnl) |
| 394-for | ATCGGTACCTGAACGTTACAGAAAAGCAGG (Kpnl) |
| 1563-rev | ACTACTAGTGCCTCTTCGCTATTACGCCAGC (Spel) |
| 1674-rev | AGAACTAGTGGAAGATCGCACTCCAGC (Spel) |
| 1451-rev | ACAACTAGTAACTTTTCGGCCAATGGTCTTG (Spel) |
| 1366-rev | ACTACTAGTCCTATAGTTTTTTCTCCTTGACGTTAAA (Spel) |

### (ii) FOA-Behandlung von S. cerevisiae Y190

Für das erfindungsgemäße Verfahren werden Reportergene benötigt, die in das Genom der Hefe integriert sind. Hierzu muss ein Selektionsmarker zur Verfügung stehen, so dass nur Transformanten, die tatsächlich das gewünschte Gen am richtigen Locus in das Genom integriert haben, wachsen können.

Der Hefestamm Y190 braucht also zusätzlich zu den Auxotrophie-Markern Leucin und Tryptophan, die durch das Two-Hybrid-System belegt sind, noch einen zusätzlichen Marker. Hierfür bietet sich Uracil (URA3-Gen) an, da bei diesem Gen die Möglichkeit besteht, den Stamm für diese Substanz auxotroph zu machen.

Bei der Herstellung URA3 negativer Klone wird die natürliche Mutationsfrequenz der Hefe von etwa 10⁻⁴ zunutze gemacht. Um bei den Hefen auf die Mutationsereignisse selektionieren zu können, wird ein Medium verwendet, das FOA (5-fluoro-orotic acid) enthält (Treco DA, 1989). Hefezellen, die kein Uracil mehr produzieren, also den gewünschten Phänotyp aufweisen, überleben, während die Zellen ohne Mutation im URA3-Gen absterben (Boeke et al., 1984).

Die bei diesem Prozess erhaltenen Kolonien wurden auf alle Marker überprüft, bevor eine Hefe dann als Ausgangspunkt der folgenden Experimente dient (Y190D).

### (iii) RedStar

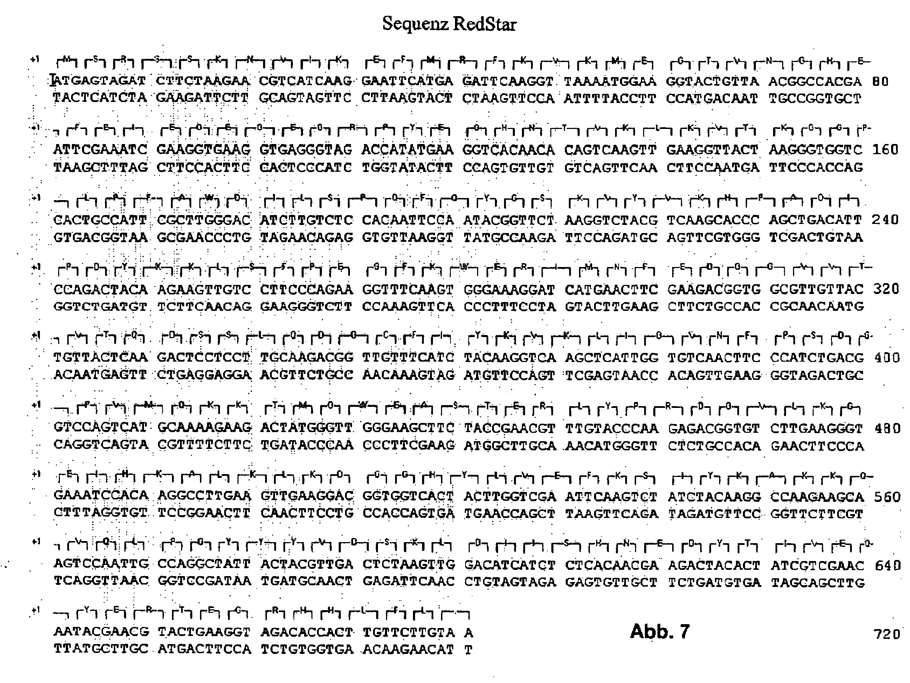

RedStar (RFP) ist ein für die Nutzung in *Saccharomyces cerevisiae* optimiertes Fluorophor (Knop et al., 2002, s. Abb. 7).

Zum GAL-Promotor und dessen Amplifizierung aus dem Genom von Y190 s. Abschnitt (i).

Die Amplifikation von RedStar erfolgte mit folgenden Primern:
RedStar-for ACTACTAGTTATGAGTAGATCTTCTAAGAACGTC (Spel)
RedStar-rev TATTCCGCGGTTACAAGAACAAGTGGTGTCTAC (Sacll)
Der jeweilige Promotor und das FtedStar-Gen wurden in einer Drei-Fragmente-Ligation (25 fmol Vektor, 125 fmol Inserts) in pRS306 kloniert. Hierbei handelt es sich um einen integrationsvektor. Auf die integration von RedStar (oder aller anderen Reportergene unter der Kontrolle des GAL-Promoters) ins Genom der Uracilauxotrophen Hefe Y190D (s. (ii)) kann mittels des Uracil-Markers von pRS306 selektiert werden.

### (iv) cob A

cob A codiert für Uroporphyrinogen III Methyltransferase aus *Propionibacterium freudenreichii.* Überexpression dieses Gens führt zu einer Fluoreszenz im Bereich von 605 nm, die auf die Akkumulation des fluoreszenten Produktes Trimethylpyrrocorphin beruht (Wildt und Deuschle, 1999).

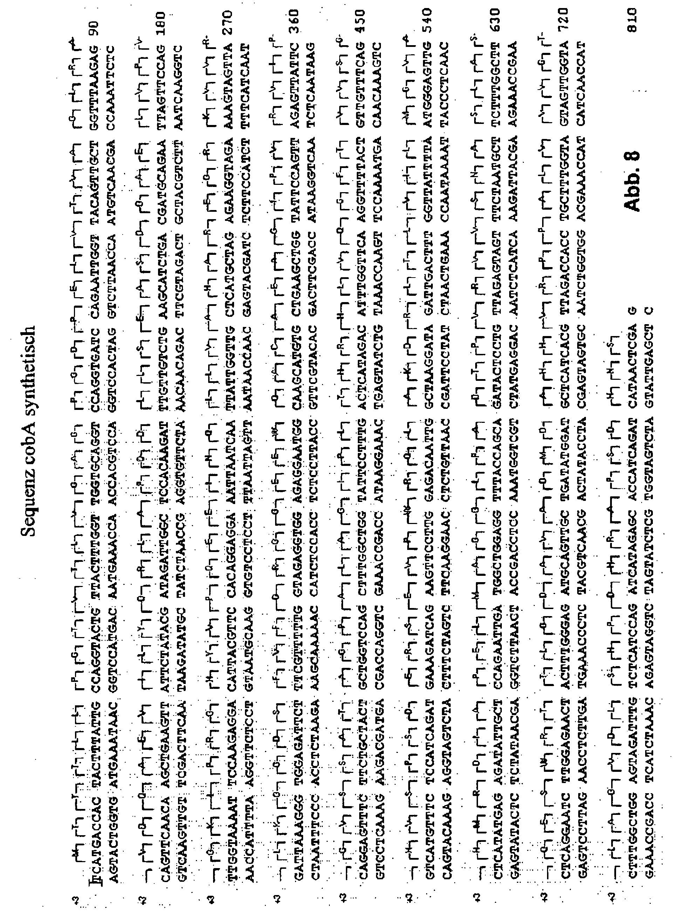

Eine Analyse des Codon Usage ergab einen hohen Prozentsatz an kritischen Codons bei der Expression des bakteriellen Gens in Hefe. Daraufhin wurde die Sequenz auf die Häufigkeit der Codonnutzung (Codon Usage) von *S. cerevisiae* optimiert und synthetisiert (Sequenz s. Abb. 8)

Auch hier wurde das Gen nach dem Anfügen eines His-Tags und einer Terminationssequenz (s.o.) zusammen mit dem Promotor der Wahl (s.o.) über Sacl/Notl in pRS306 kloniert und in das Genom der Hefe integriert.

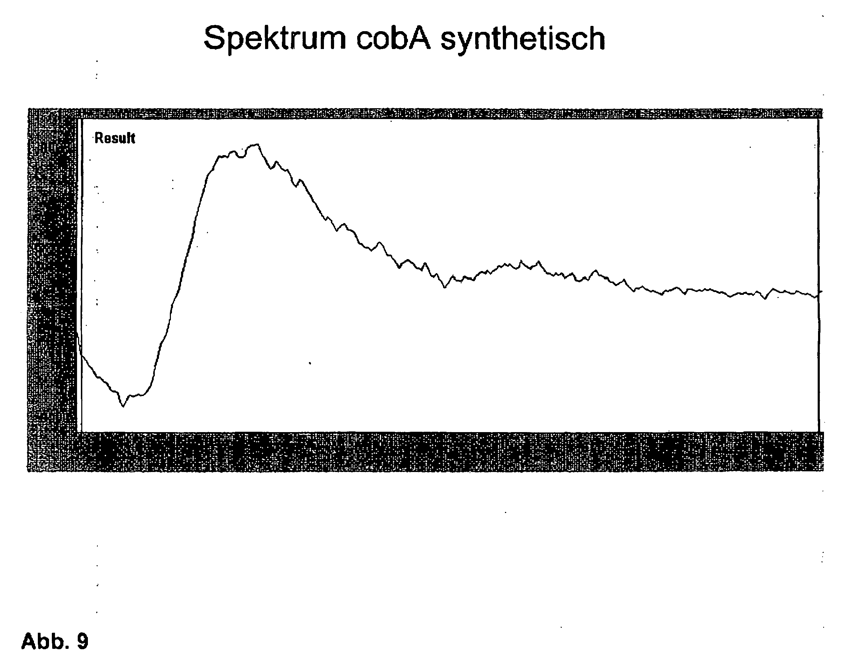

Es ist eine deutliche Fluoreszenz der Hefekolonien festzustellen. Auch ein zur weiteren Absicherung bei der Anregungswellenlänge von 540 nm aufgenommenes Emissionsspektrum beweist die erfolgreiche Entwicklung im Hinblick auf das erfindungsgemäße Verfahren (Spektrum s. Abb. 9, Differenzspektrum aus cobA exprimierender Hefe und Hefe ohne Reportergenexpression).

### (v) Met 1

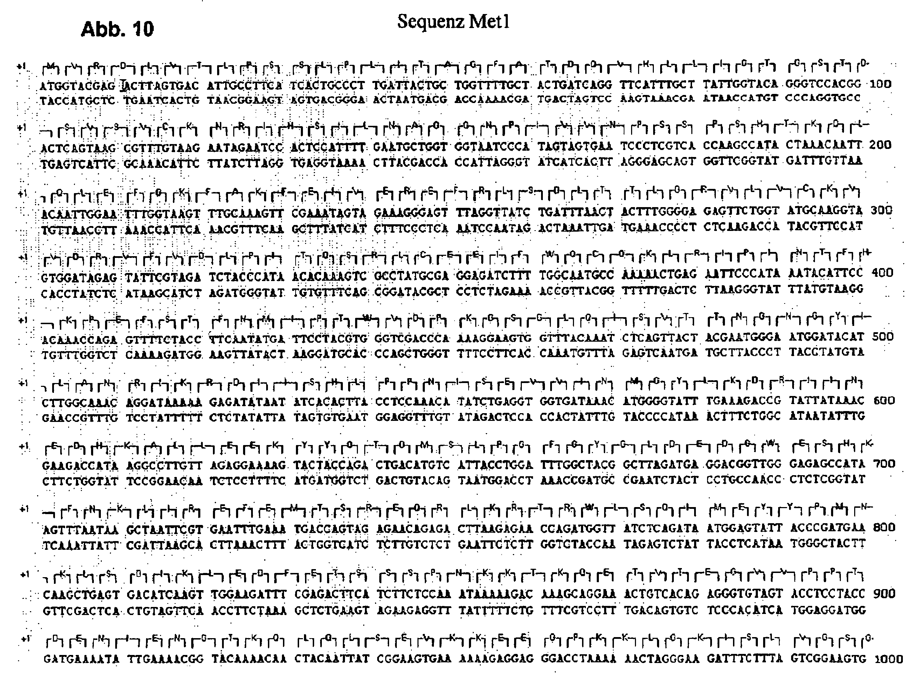

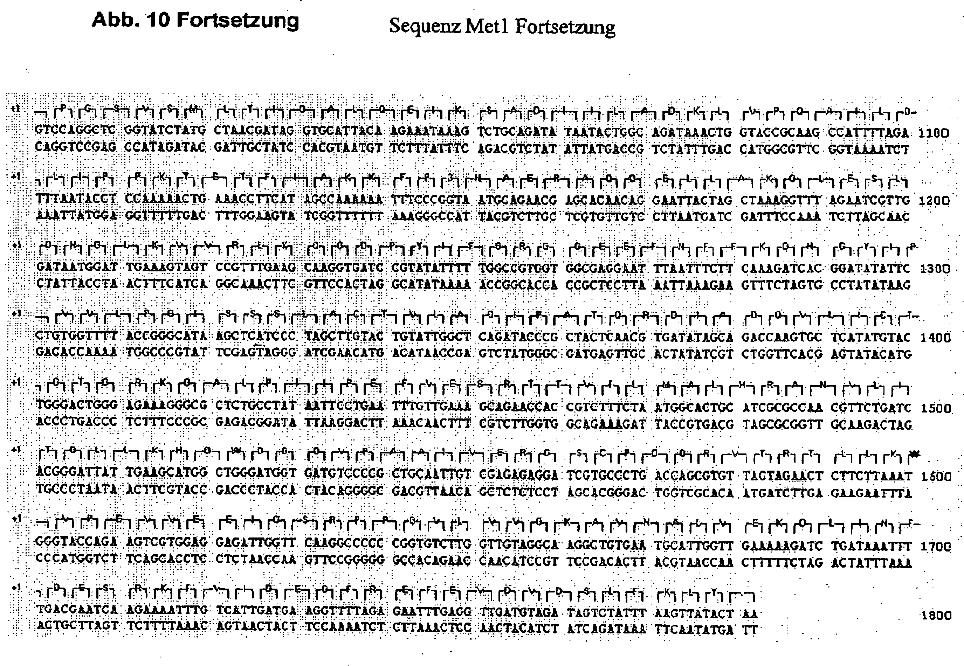

MET1 ist das entsprechende Protein aus *Saccharomyces.* Das 1,8 kb große Gen (Sequenz s. Abb 10) wurde aus dem Hefegenom mittels PCR amplifiziert.

### Eingesetzte Primer:

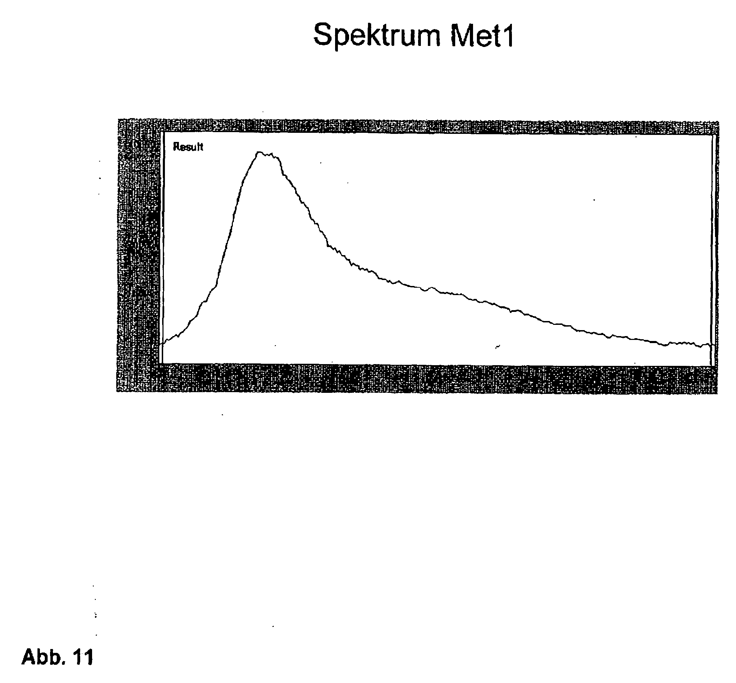

Met1-for: AATTATCCATGGTACGAGACTTAGTGACATTG (Ncol)
Met1-rev:
   AATTAACTCGAGTTGTATAACTTAAATAGACTATCTACATCAACC (Xhol)
Das Fragment wurde über Ncol/Xhol (Ncol enthält das Startcodon) in einen Vektor kloniert, der das Anfügen eines His-Tags und einer Terminationssequenz für Hefegene erlaubt (Amtz et al., 2004). Nach der Klonierung des Reportergens (NcoI/NotI) mit dem Promotor der Wahl (Sacl/Ncol) über SacI/NotI in den Vektor pRS306 erfolgte die Integration des Reportergens in das Genom der Hefe. Auch dieses Reportergen für das erfindungsgemäße Verfahren konnte erfolgreich exprimiert werden. Im Emissionsspektrum ist bei einer Anregung von 550 nm der spezifische Peak bei ca. 600 nm (s. Abb 11, Diefferenzspektrum aus Met1 exprimierender Hefe und Hefe ohne Reportergenexpression) am größten.

### b) Durchführung des quantitativen Screenings im erfindungsgemäßen Verfahren

Zu der Durchführung des quantitativen Screenings gehören die Herstellung des Mediums, die Transformation der Hefen und das Scannen der Platten. Dabei müssen alle Parameter standardisiert und optimiert sein, damit die Fluoreszenzergebnisse reproduzierbar sind.

### (i) Herstellung des Mediums

Für die Kultivierung und das Scannen der Hefen auf Fluoreszenz durch den LSA-Scanner werden folgende Medien benötigt:
**YPAD-Medium** (Vollmedium für Hefen)
5.0 g Yeast extract (Difco)
10.0 g Peptone (Difco)
50 mg Adenine hemisulphate
ddH₂O ad 460 ml
pH vor dem Autoklavieren auf 5.8 einstellen; nach dem Autoklavieren hat das Medium einen pH-Wert von 5.6;
Für Agar-Platten: Zusatz 10 g Yeast-agar (Difco) nach der pH-Einstellung Autoklavieren 15 Minuten bei 121 °C;
Nach dem Autoklavieren werden 40 ml 25%ige Glucose (wird getrennt vom Medium autoklaviert) hinzugefügt.

### Synthetisches Komplettmedium (ohne Leu, Trp, His)

3.35 g Yeast Nitrogen Base (w/o amino acids)
1 g Synthetic Complete Drop Out Mix (Aminosäuremix ohne Leu. Trp, His) ddH₂O ad 460 ml;
pH vor dem Autoklavieren auf 5.8 einstellen; nach dem Autoklavieren hat das Medium einen pH-Wert von 5.6;
Für Agar-Platten- Zusatz 10 g Yeast-agar (Difco) nach der pH-Einstellung Autoklavieren 15 Minuten bei 121 °C;
Nach dem Autoklavieren werden 40 ml 25 %ige Glucose (wird getrennt vom Medium autoklaviert) und 10 ml 2.5 M 3-Amino-1,2,4-Triazol (sterilfiltriert) hinzugefügt.
Endkonzentration Glucose im Medium: 2 %

### 25% ige Glucose

100 g Glucose (Sigma)
400 ml ddH₂O
Autoklavieren 15 Minuten bei 121 °C

### 2.5 M 3-Amino-1,2,4-Triazol

1.051 g 3-Amino-1,2,4-Triazol
5 ml ddH₂O
Mit einem Sterilfilter (Durchmesser 0,45 µm) filtrieren;
Die Endkonzentration 3-Amino-1,2,4-Triazol im Medium variiert je nach Experiment zwischen 0 und 50 mM,

Standardmäßig werden Omnitray-Platten (Firma Nunc), auf denen die Hefen für das Scannen im LSA-Scanner kultiviert werden, mit einem Volumen von 78 ml Medium gegossen. Hierdurch entsteht immer eine gleiche Scanhöhe für den Scanner, die auf 9,9 mm eingestellt ist.

### (ii) Durchführung der Hefe-Transformation

Folgendes auf die höchstmögliche Transformationseffizienz optimierte Protokoll kommt zur Anwendung.

20-30 ml flüssiges YPAD (Vollmedium) werden mit den zu transformierenden Hefen (Y190D mit integriertern Reportergen) beimpft und über Nacht bei 30°C und 200 rpm inkubiert. Am nächsten Tag werden zu 50 ml YPAD (auf Raumtemperatur aufgewärmt) Hefen von der Vorkultur hinzupipettiert bis eine OD₆₀₀ von etwa 0,05 erreicht ist. Die Kultur wird bei 30 °C und 150-200 rpm inkubiert, bis eine Zelldichte von 2x10⁶ - 4x10⁶ Zellen/ml erreicht ist. Dieses entspricht einer OD₆₀₀ von 0,2 - 0.4 (dauert ca. 3-5 h). Die Kultur wird in einem sterilen 50 ml Zentrifugen-Röhrchen bei 3000 x g (3500 rpm in der Hettich-Zentrifuge) und 5 Minuten geerntet. Das Medium (Überstand) wird entfernt und die Zellen werden in 25 ml sterilem ddH₂O resuspendiert. Nach dem Resuspendieren wird erneut 5 Minuten bei 3000 x g zentrifugiert (3500 rpm Hettich-Zentrifuge). Der Überstand wird entfernt und die Zellen werden in 1,0 ml 100 mM LiAc resuspendiert. Die Suspension wird in ein 1,5 ml Eppendorfcup überführt. Nun werden die Zellen 15 Minuten bei 30°C inkubiert. Anschließend werden die Zellen durch Zentrifugation "full speed" für 15 Sekunden pelletiert und der Überstand abpipettiert. Diese Menge an Zellen reicht für einen Transformationsansatz. Wenn 2 Transformationsansatze gemacht werden sollen, müssen 100 ml (2 x 50 ml) kompetente Zellen hergestellt werden und mit Lithiumacetat vorbehandelt werden. Zu den ZeHen wird der folgende "Transformationsmix" in der angegebenen Reihenfolge pipettiert:
X µl Plasmid DNA (0,1-10 µg)
34-X µl Steriles ddH2O
(Zellen in Wasser + Plasmid-Lösung resuspendieren durch Auf- und Abpipettieren, dann erst PEG dazu pipettieren)
240 µl PEG (50 % w/v)
(Zellen mischen mit PEG durch kurzes Vortexen)
36 µl 1,0 M LiAc
50 µl ss-DNA (2,0 mg/ml)
360 µl totales Volumen
Die Zellen werden kräftig gevortext, bis eine homogene Suspension entstanden ist (ca. 1 min). Der Transformationsansatz wird bei 30 °C 30 min im Schüttler (800 rpm) inkubiert und anschließend bei 42°C ins Wasserbad gestellt (Heat-Schock). Nach den Inkubationen wird der Transformationsansatz 15 Sekunden bei 6-8000 rpm zentrifugiert und der Transformationsmix mit einer Eppendorfpipette aus dem Eppendorfcup entfernt. Zu dem Pellet (Zellen) wird 1,0 ml steriles ddH₂O gegeben und das Pellet wird durch langsames Auf- und Abpipettieren resuspendiert. Schnelles Auf- und Abpipettieren verringert die Transformationseffizienz. Die aufgelösten transformierten Zellen werden einmal 1:100 und einmal 1:10.000 verdünnt und zwischen 2 und 200 µl der verdünnten Zellen werden auf SC-Medium ohne Leucin. Tryptophan und Histidin mit einer geeigneten Konzentration an 3-Aminotriazole ausgestrichen. Die Anzahl der zu erwartenden Kolonien liegt bei einem ausplattierten Volumen von 20 - 200 µl von einer Verdünnung von 1:10.000 bei 0 - 50 Kolonien pro Platte und bei einem ausplattierten Volumen von 10 - 200 µl von einer Verdünnung von 1:100 bei 200 - > 5000 Kolonien pro Platte. Die Transformationseffizienz liegt dann bei 500.000 - 2.000.000 Zellen pro µg Plasmid-DNA (bei steigender 3-AT Konzentration nimmt die Transformationseffizienz ab). Die Platten werden 2 - 6 Tage bei 30°C bebrütet.

Nach 2 - 6 Tagen (je nach Interaktionspaar und 3-AT-Konzentration) können die Hefezellen im Scanner LS-400 (Tecan) gescannt und ausgewertet werden.

### (iii) Scannen mit dem Tecan LS-400

Die Hardware (der Scanner Tecan LS-400) mit passender Software ist bei Tecan erhältlich.
Die Höhe des Agars (Scanhöhe) der Omnitray Platte beträgt mindestens 8,0 mm.
Es wurden für die Messungen fertige Methoden angelegt. Klone mit Redstar, cobA oder Met1 im Genom und Two-Hybrid- oder n-Hybrid-Plasmiden werden mit dem Laser 543 nm und dem Filter 590 nm (Bandpass 20 nm) gescannt.
Die Aufnahmen wirden nicht-konfokal durchgeführt.
Scan Resolution (Bildauflösung) ist beim Scannen einer normal gewachsenen Kultur (Durchmesser ca. 1-2 mm) auf 20 µm eingestellt. Wenn eine Kultur aus kleineren Kolonien besteht, wird die Scan Resolution auf 4 bis 8 µm herunter gesetzt.

### (iv) Auswertung der gescannten Kolonien mit Optimate

Zum Auswerten von der Messungen wird die Software Optimate benutzt, die für diese Anwendung in Kooperation entwickelt wurde und kommerziell bei Tecan erhältlich ist.
Folgende Einstellungen sind optimiert für eine Kultur, die aus Kolonien mit einem Durchmesser von 1-2 mm besteht:
Minimum Object: 70
Roundness: 15.2
Threshold Power: 15
Alle Kolonien, die einzeln liegen und groß genug sind, werden ausgewertet. Die Fluoreszenzintensität wird auf die Fläche normiert.

### c) Klonierung des Kompetitors

### (i) Wahl verschiedener Promotoren für den Kompetitor - Vortest

Die Konzentration des Kompetitors ist wesentlich für das n-Hybrid System. Je mehr Genprodukt vorhanden ist, desto mehr verschiebt sich das Gleichgewicht auf die Seite des inaktiven Komplexes aus Kompetitor und Fusions-Köderprotein.

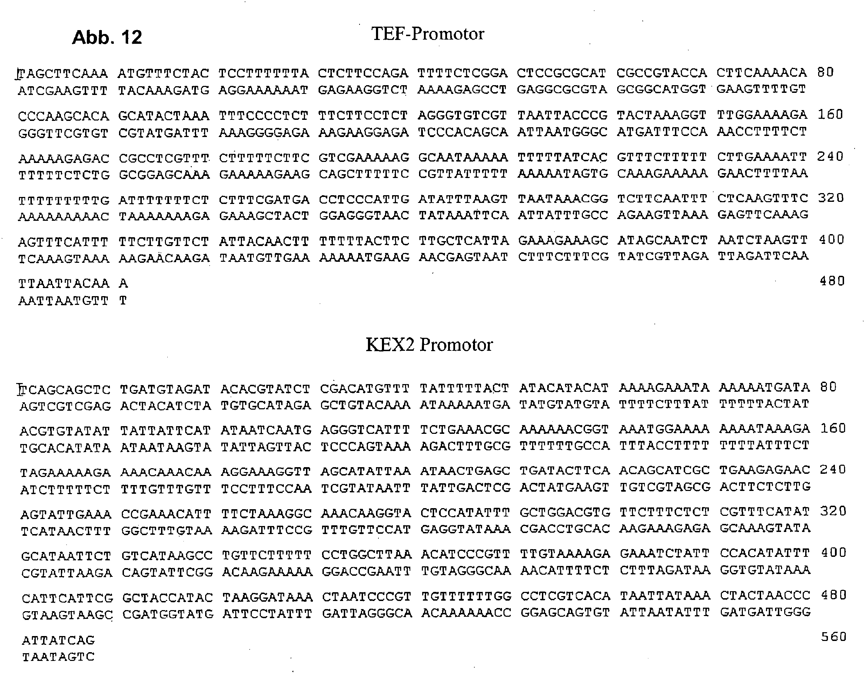

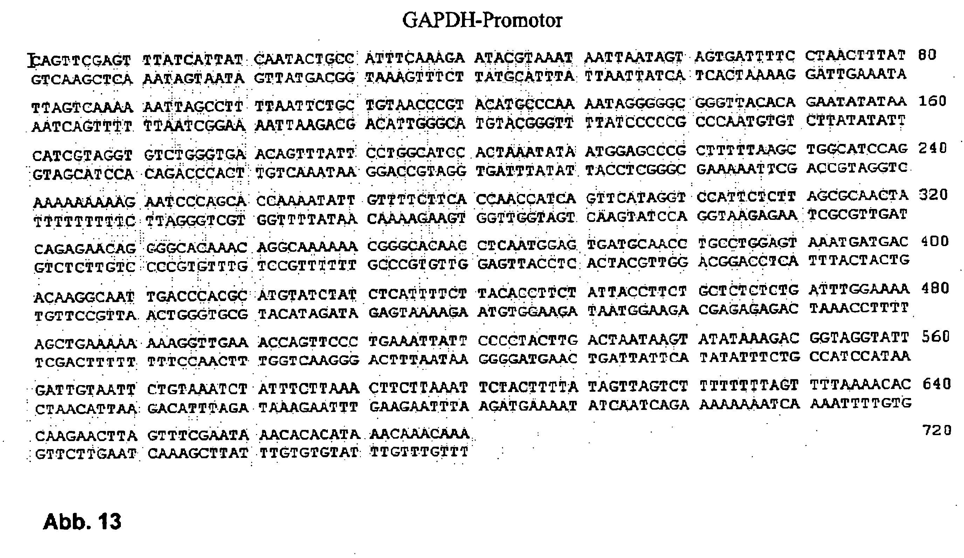

Um variable Konzentrationen des Kompetitors zu gewährleisten, sollten verschiedene, in der Literatur (Nacken et al, 1996) als konstitutiv beschriebene Promotoren zur Expression des Kompetitors eingesetzt und in unserem Hefestamm validiert werden.
**KEX2** (Fuller et al. 1989. M24201), Sequenz s. Abb. 12, 488 bp
KEX2-for: ATCCTTGAGCTCTCAGCAGCTCTGATGTAGATACAC (Sacl)
KEX2-rev:
ATCCCCCATGGCTGATAATGGGTTAGTAGTTTATAATTATGTG (Ncol)
**TEF** (Cottrelle et al., 1985, M10992) Sequenz s. Abb. 12 , 411 bp
TEF-for: ATCCCCGCGGTAGCTTCAAAATGTTTCTACTCC (Sacll)
TEF-rev: ATCCCCCATGGTTTGTAATTAAAACTTAGATTAGATTG (Ncol)
**GAPDH** (Bitter und Egan, 1984, M13807), Sequenz s. Abb.13, 680 bp
GAPDH-for: ATCCCCGCGGCAGTTCGAGTTTATCATTATCAATAC (Sacll)
GAPDH-rev: ATCCCCCATGGTTTGTTTGTTTATGTGTGTTTATTC (Ncol)

Diese Promotoren wurden mit den angegebenen Primern aus dem Hefe-Genom amplifiziert (Sacll bzw. Sacl/Ncol), mit dem RedStar-Gen (BspHI/Notl) zusammen in pRS306 (Sacll bzw. Sacl/Notl) kloniert und in das Hefegenom integriert. Eine Bestimmung der Fluoreszenzintensität der Hefekolonien im Two-Hybrid-System mittels quantitativem Screening ergab folgende Reihenfolge der Promotorstärke: GAPDH > TEF > KEX2: dabei ist KEX2 als sehr schwacher Promotor zu bezeichnen. Dieses Ergebnis bestätigt die Voreinschätzung laut Literatur.
Alle drei Promotoren wurden im Folgenden vor den Kompetitor kloniert (s. folgenden Abschnitt).

### (ii) Klonierung des Beuteprotein-Kompetitors auf das Fusions-Köderprotein-Plasmid

Für das erfindungsgemäße Verfahren wird der Kompetitor in eines der beiden Two-Hybrid-Plasmide kloniert und so idealerweise, wie das Köder- und Beuteprotein, von der Zelle selbst synthetisiert. Soll das Beuteprotein als Kompetitor verwendet werden, wird dieses auf den Vektor mit dem Fusions-Köderprotein kloniert; soll das Köderprotein als Kompetitor vorliegen, wird dieses auf den Vektor mit dem Fusions-Beuteprotein kloniert, Hierdurch werden mögliche Rekombinationen zwischen gleichen Gensequenzen, die in der Hefe stattfinden können, vermieden. Als Ausführungsbeispiel soll die Klonierung von RafRBD (Beuteprotein-Kompetitor) auf pPC97-ras (Fusions-Köderprotein-Plasmid) beschrieben werden.

In pPC97-ras liegt folgende Struktur vor:

Promotor (ADH) - GAL4-BD - ras - mcs (AatII/SacI/SacII) - Terminator (ADH)

Um den Kompetitor einklonieren zu können, muss ein Terminator nach dem ras-Gen eingefügt werden; dann soll der Promotor und dann der Kompetitor folgen. Hierzu wird der Terminator verwendet, der auch an sonstige zu klonierende Gene angehängt wird (s. Arntz et al., 2004). In diesem Fall werden zwei Oligos annealt (Term-Raf-for und -rev; Sequenz s.u.). Hierzu werden die Oligos in einer Endkonzentration von je 2 pmol/µl in einer PCR-Maschine (94 °C 2 min, 70 x -1 °C, jeweils 1 min bei dieser Temperatur, 4 °C; Angabe der Firma Pierce: Anneal complementary pairs of oligonucleotides, Technical Resource) annealt. Für die anschließende Ligation in den Vektor werden 2 µl eingesetzt.
Die Klonierung in das Fusions-KÖderprotein-Plasmid erfolgt über Aatll/Sacl. Das RafRBD-Gen wird amplifiziert (PciI/SacII) und zusammen mit dem jeweiligen Promotor der Wahl (Sacl/Ncol) in einer Drei-Fragmente Ligation in den Vektor mit Terminator kloniert (SacI/SacII). Es ergibt sich die aus Abbildung 5 ersichtliche Struktur. Verwendete Primer:
Term-Raf-for: CTATATAACTCTGTAGAAATAAAGAGTATCATCTTTCAAAGAGCT
Term-Raf-rev:
   CTTTGAAAGATGATACTCTTTATTTCTACAGAGTTATATAGACGT
RafRBD-Pci-for: AATTCCACATGTCCGACCCGAGTAAGACAAGC (Pcil)
RafRBD-Sacll-rev:
   ATTGCCGCGGTTAGTCGACATCTAGAAAATCTACTTGAAG (Sacll)

### 2. Grenzen des bekannten Zwei-Hybrid-Systems

Im dem bekannten Zwei-Hybrid System wurden die Reportergenaktivitäten der RafRBD-Mutanten R67A, T68A, V69A und A85K sowie des Wildtyps untersucht. Diese Mutanten unterscheiden sich bekanntlich in ihren Bindungsaffinitäten, wobei sie sich wie folgt nach steigender Bindungsaffinität ordnen lassen:

RafRBD-R67A < T68A < V69A < WT < A85K

Werden diese Mutanten unter den bei Jaitner et al, (1997) beschriebenen Expressionsbedingungen in dem bekannten Zwei-Hybrid System untersucht, bestätigt sich diese Rangfolge. Im Einzelnen ergeben sich folgende Werte:

**Tab 1: Reportergen-Aktivität bei Verwendung von Met1 als Reportergen; die Reportergen-Aktivität ist als % der Wildtyp Aktivität dargestellt.**

| RafRBD-Mutante | Reporter Fluoreszenz (willkürliche Einheit) | Reporter Aktivität in Relation zu Wildtyp (WT) |
|---|---|---|
| RafRBD-R67A | 5196 | 62% |
| RafRBD-T68A | 5940 | 70 % |
| RafRBD-V69A | 6975 | 83 % |
| RafRBD-wt | 8430 | 100 % |
| **RafRBD-A85K** | **9056** | **107 %** |

Anhand des Vergleichs der RafRBD-85K Mutante mit dem Wildtyp im Zwei-Hybrid-System ergibt sich daher die Schlussfolgerung, daß die Mutante lediglich eine um 7% erhöhte Bindungsaktivität aufweist. Aus mikrokalorimetrischen Messungen ist jedoch bekannt, daß diese Mutante im Vergleich zum Wildtyp eine deutlich höhere Affinität zeigt; nämlich eine Dissoziationskonstane von 72 nM Vergleich zu 253 nM des Wildtyps (siehe oben und Abb. 4). Damit wird deutlich, daß das bekannte Zwei-Hybrid System nicht dazu geeignet ist, hochaffine Mutanten vom Wildtyp abzugrenzen und darüber zu identifizieren. Die Identifizierung eines hochaffinen Proteins ist nämlich in der Praxis nur dann möglich, wenn die höhere Affininität des mutierten Proteins zu einer deutlichen und zweifelsfreien Diskriminierung gegenüber der Wildtyp-Form über den Read-out des Verfahrens (hier: Reportergen-Aktivität) führt.

### 3. Einfluß des Promotors auf die Bestimmung der Reportergen-Aktivität im erfindungsgemäßen Verfahren mit Kompetitor.

Das erfindungsgemäße Verfahren kann zur Bestimmung des von der Untersuchung erfassten dynamischen Bereichs - d.h. zur Optimierung des Selektionsergebnisses - unter anderem durch die Konzentration des Kompetitors variiert werden. Die Konzentration des in der Wirtszelle exprimierten Kompetitors kann dabei beispielsweise durch die Wahl des dem Kompetitor vorgeschalteten Promotors kontrolliert werden (s. 2c) Klonierung des Kompetitors).

Die Möglichkeiten der Beeinflussung des erfindungsgemäßen Systems über den Promotor des Kompetitors zeigen Versuche mit den unterschiedlich starken Promotoren KEX2, TEF und GAPDH. Von diesen drei Promotoren ist KEX2 der schwächste, während GAPDH der stärkste ist (s. 2c) Klonierung des Kompetitors).

Bereits der Einsatz des schwächsten Promotors (KEX2) zur Expression des Kompetitors führt zu einer deutlich verbesserten Abgrenzung der in ihrer Affinität erhöhten Mutante RafRBD-A85K gegenüber dem Wildtyp Protein. So wird unter Verwendung von Met1 als Reporter im Vergleich zu der Wildtyp-Form eine Aktivität von 120% (Tab.2) und unter Verwendung von RedStar als Reporter von 131% (Tab.3) gemessen. Dies stellt gegenüber der nachweisbaren Aktivität von 107% im herkömmlichen Zwei-Hybrid System (siehe oben) tatsächlich eine Basis dar, auf deren Grundlage in der Praxis Protein-Interaktionen mit erhöhter Bindungsaktivität nachgewiesen und damit höher affine Proteine identifiziert werden können.

**Tab 2: Reportergen-Aktivität bei Verwendung von Met1 als Reporter und unter Expression des Kompetitors RafRBD-wt unter dem KEX2 Promotor; die Reportergen-Aktivität ist als % der Wildtyp Aktivität dargestellt.**

| RafRBD-Mutante | Reporter-Fluoreszenz (willkürliche Einheit) | Reporter-Aktivität in Relation zu WT |
|---|---|---|
| RafRBD-R67A | 3264 | 41 % |
| RafRBD-T68A | 5343 | 67 % |
| RafRBD-V69A | 6651 | 83 % |
| RafRBD-wt | 7969 | 100 % |
| **RafRBD-A85K** | **9545** | **120 %** |

**Tab 3: Reportergen-Aktivität bei Verwendung von RedStar als Reporter und unter Expression des Kompetitors RafRBD-wt unter dem KEX2 Promotor; die Reportergen-Aktivität ist als % der Wildtyp-Aktivität dargestellt.**

| RafRBD-Mutante | Reporter-Fluoreszenz (willkürliche Einheit) | Reporter-Aktivität in Relation zu WT |
|---|---|---|
| RafRBD-R67A | 4514 | 40% |
| RafRBD-T68A | 5737 | 51 % |
| RafRBD-V69A | 7889 | 69 % |
| RafRBD-wt | 11353 | 100 % |
| **RafRBD-A85K** | **14857** | **131 %** |

Bei Einsatz des stärkeren TEF Promotors zur Expression des Kompetitors wird die Erhöhung der RafRBD-A85K induzierten Met1-Reportergen-Aktivität gegenüber dem Wildtyp noch weiter verstärkt (Tab.4). So zeigt dieser Reporter in dem erfindungsgemäßen Verfahren unter Verwendung des TEF Promotors eine Aktivität, die im Vergleich zum Wildtyp bei 137 % liegt (bei Verwendung des KEX2 Promotors lag die Aktivität bei nur 120%; Tab. 2). Wird dagegen RedStar als Reporter eingesetzt, liegt die Reportergen-Aktivität gegenüber dem Wildtyp bei 139% (Tab. 5).

**Tab 4: Reportergen-Aktivität bei Verwendung von Met1 als Reporter und unter Expression des Kompetitors RtafRBD-wt unter dem TEF Promotor; die Reportergen-Aktivität wird als % der Wildtyp Aktivität dargestellt.**

| RafRBD-Mutante | Reporter Fluoreszenz (willkürliche Einheit) | Reporter Aktivität in Relation zu WT |
|---|---|---|
| RafRBD-R67A | 1510 | 68 % |
| RafRBD-T68A | 1600 | 72 % |
| RafRBD-V69A | 1662 | 75 % |
| RafRBD-WT | 2213 | 100 % |
| **RafRBD-A85K** | **3031** | **137 %** |

**Tab 5: Reportergen-Aktivität bei Verwendung von RedStar als Reporter und unter Expression des Kompetitors RafRBD-WT unter dem TEF Promotor; die Reportergen-Aktivität ist als % der Wildtyp Aktivität dargestellt.**

| RafRBD-Mutante | Reporter Fluoreszenz (willkürliche Einheit) | Reporter Aktivität in Relation zu WT |
|---|---|---|
| RafRBD-R67A | 2747 | 30 % |
| RafRBD-T68A | 4334 | 47 % |
| RafRBD-V69A | 9095 | 99% |
| RafRBD-wt | 9233 | 100 % |
| **RafRBD-A85K** | **12873** | **139 %** |

Das erfindungsgemäße Verfahren wurde auch unter Verwendung des starken GAPDH Promotors zur Expression des Kompetitors getestet. Mit der Wahl dieses Kompetitors war für die RafRBD-A85K-Variante wiederum eine Erhöhung der RedStar Reportergen-Aktivität im Vergleich zum Wildtyp zu beobachten. Diese Aktivität lag bei 168% (Tab. 6).

**Tab 6: Reportergen-Aktivität bei Verwendung von RedStar als Reportergen und unter Expression des Kompetitors RafRBD-WT unter der Kontrolle des GAPDH Promotors; die Reportergen-Aktivität ist als % der Wildtyp Aktivität dargestellt.**

| Konstrukt | Reporter Fluoreszenz (willkürliche Einheit) | Reporter-Aktivität in Relation zu WT |
|---|---|---|
| RafRBD-R67A | 1999 | 22 % |
| RafRBD-T68A | 2997 | 34 % |
| RafRBD-V69A | 7812 | 88 % |
| RafRBD-wt | 8912 | 100 % |
| **RafRBD-A85K** | **15014** | **168 %** |

### 4. erfindungsgemäßes Verfahren mit erhöhtem Selektionsdruck

Die durch das erfindungsgemäße Verfahren erfassbare relative Reportergen-Aktivität des mutierten Beute- oder Köderproteins im Vergleich zum Wildtyp-Protein kann durch den gezielten Einsatz eines Selektionsdruck auf die transformierten Wirtszellen noch erhöht werden (s. o.). So kann z.B. dem Kultivierungsmedium 3-Aminotriazol als Hemmstoff für die His-Expression hinzugefügt werden.

In einer besonders vorteilhaften Ausführungsform wird das erfindungsgemäße Verfahren unter Expression des Kompetitors RafRBD-wt unter Kontrolle des TEF-Promotors und unter Zugabe einer gegenüber den bie Jaitner et al. (1997) beschriebenen Standardbedingungen erhöhten Aminotriazol Konzentration durchgeführt. Damit wird die Diskriminierung zwischen dem Wildtyp-Protein und der in der Affinität verbesserten Mutante RafRBD-A85K nochmals verstärkt. Die Aktivität dieser Mutante im Vergleich zum Wildtyp lag bei 199% (Tab. 7)

**Tab 7: Reportergen-Aktivität bei Verwendung von RedStar als Reportergen und unter Expression des Kompetitors RafRBD-wt unter dem TEF Promotor in Abhängigkeit von der 3-AT Konzentration: die Reportergen-Aktivität wird als % der Wildtyp Aktivität dargestellt.**

| Konstrukt | Reporter Aktivität in Relation zum Wildtyp bei 25 mM 3-AT | Reporter Aktivität in Relation zum Wildtyp bei 50 mM 3-AT |
|---|---|---|
| RafRBD-T68A | 21 % | 34% |
| RafRBD-wt | 100% | 100% |
| **RafRBD-A85K** | **125%** | **199%** |

Mit Hilfe des erfindungsgemäßen Verfahrens konnte die in der Affinität zu Ras verbesserte RafRBO Mutante RafRBD-A85K somit eindeutig anhand der erhöhten Reportergen-Aktivität identifiziert werden.

5) erfindungsgemäßes Verfahren unter Einsatz von Zufallsmutagenese und robotergestütztem Hitpicking

Zum Nachweis der Funktionalität des erfindungsgemäßen Verfahrens müssen in der Bindungsaffinität gesteigerte Mutanten aus einer großen Anzahl von Fremdsequenzen selektiert werden können. Dabei muss im erfindungsgemäßen Verfahren, wie erläutert, die Diskriminierung der verbesserten Mutanten gegenüber dem Wildtyp im Vergleich zu den Ergebnissen des Two-Hybrid-Verfahrens verbessert sein.

Zu diesem Zweck wurde ein Verfahreb durchgeführt, das sich zusammensetzt aus der Erzeugung der Mutanten (Zufallsmutagenese), der Transformation der mutierten Vektoren ("Bank") in die Hefe und dem Hitpicking, in dem die am stärksten fluoreszenten Kolonien nach dem quantitativen Screening durch den Roboter (Tecan Genesis Freedom) selektiert wurden. Angeschlossen an diesen Prozess wird die Isolation der Plasmid-DNA aus den Hefen, die Transformation dieser DNA in Bakterien (beide Vorgänge robotergestützt), die Sequenzierung und letztendlich die Auswertung der erhaltenen Mutanten.

Als Beispiel für die Generierung von Mutanten mit erhöhter Affinität wurden Zufallsmutagenesen anhand des Interaktionpaares Ras/RafRBD durchgeführt.

### a) Zufallsmutagenese

Für die Zufallsmutagenese stehen verschiedene Methoden zur Verfügung (Neylon 2004).
Error Prone PCR (epPCR)
Die Vorteile der epPCR sind vor allem ihre universelle Einsetzbarkeit und leichte Durchführbarkeit.
Bei der epPCR, wie auch bei den anderen Methoden, bei denen das Kopieren von DNA absichtlich gestört wird (z.B. Verwendung von Mutator-Stämmen wie XL1-Red von Stratagene und Einsatz von chemischen und physikalischen Mutagenen) erfolgt die Verteilung der Mutationen zufällig über das ganze Ziel-Gen. Es werden auch Methoden beschrieben, die das gesamte Plasmid mit einer bestimmten Rate mutagenisieren (rolling circle amplification, Fujii et al. 2004). Die Erhöhung der Fehlerrate der verwendeten Taq-Polymerase erfolgt z.B. durch Einsatz von Mn2+, unausgewogenen Mengen an dNTPs oder Nukleosidtriphosphat-Analoga (Zaccolo et al. 1996). Außer diesen Möglichkeiten werden zwei Kits angeboten, die zum einen auf Veränderungen der Mn²⁺- und GTP-Konzentrationen beruhen (Diversify PCR Random Mutagenesis Kit, Clontech) bzw. zum anderen eine hoch fehleranfällige Polymerase verwenden und die Template-Konzentration variieren (GeneMorph, Stratagene).

Die epPCR als solches beruht entweder auf einer Einfügung einer falschen Base und/oder auf dem Fehlen der Korrekturlesefähigkeit der Polymerase. Die inhärente Eigenschaft der verwendeten Polymerase bedeutet, dass einige Fehler häufiger auftreten als andere. Damit treten dann einige Mutationen (wie z.B. Transitionen) häufiger auf als andere und die Bank hat eine nicht-zufällige Beschaffenheit (error bias). Mit der Kombination zweier verschiedener Methoden, bei denen verschiedene "biases" auftreten, wie der Einsatz der Taq-Polymerase und des GeneMorph Kits kann der "bias" der Bänke reduziert werden.

Weiterhin tritt der sogenannte "codon bias" auf, der auf der Natur des genetischen Codes beruht. Einfache Punktmutationen führen zu einem bias in den Varianten an Aminosäuren, die die mutierte DNA codiert. Z.B. führt eine Punktmutation in einem Valin-Codon nur zu sechs anderen Aminosäuren (Phe, Leu, Ile, Ala, Asp, Gly). Zur Codierung der anderen AS sind entweder zwei Punktmutationen (C, S, P, H, R, N, T, M, E, Y) oder sogar drei (Q, W, K) erforderlich.

Der letzte "bias" ist der "amplification bias". Er ist bei jedem Mutageneseprotokoll zu beobachten, das einen Amplifizierungsschritt beeinhaltet. Ein Molekül, das früh im Amplifizierungsprozess kopiert wurde, ist in der endgültigen Bank überrepräsentiert. Dieses Problem kann, zumindest teilweise, durch eine Kombination von verschiedenen getrennt durchgeführten epPCRs und/oder durch eine Reduktion der PCR-Zyklenanzahl überwunden werden.

Eine Eigenschaft der epPCR ist auch, dass nicht alle Basen für eine Mutagenisierung zugänglich sind und dass, statistisch gesehen, eine gegebene Aminosäure nur zu weniger als fünf anderen Aminosäuren mutagenisiert wird. (Wong et al. 2004)

### Oligonucleotid-basierte Methoden

Im Gegensatz zu der epPCR, bei der eine längere DNA-Sequenz zufällig mutagenisiert wird, haben Oligonukleotid-basierte Methoden das Ziel, nur einzelne, bestimmte Positionen des Ziel-Gens zu randomisieren. Alle Techniken basieren auf der Inkorporierung einer synthetischen DNA-Sequenz (Oligonukleotid), die unterschiedlich stark mutagenisiert sein kann, in die codierende Sequenz. Dabei kann es sich um ein Oligonucleotid oder mehrere Primer gleichzeitig handeln.

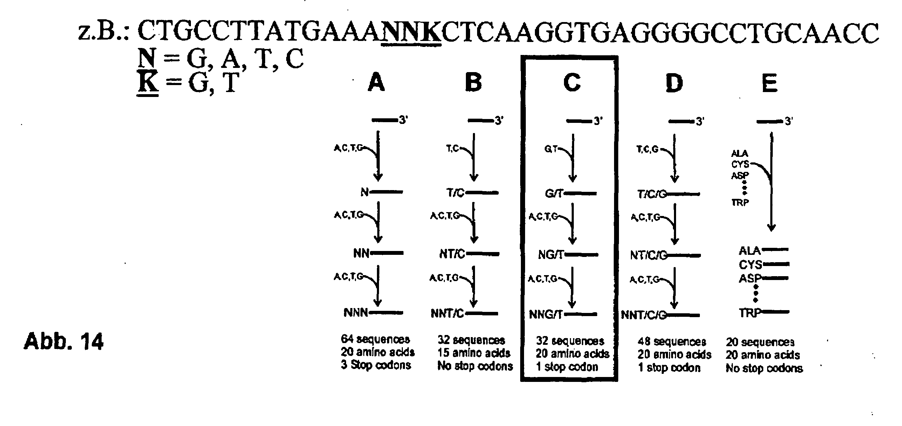

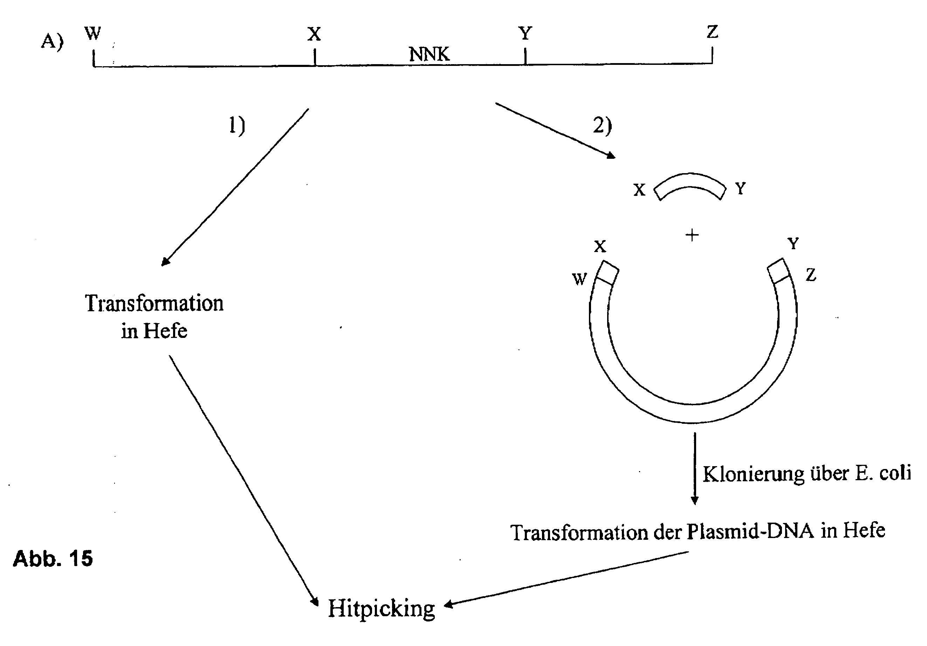

Damit alle Aminosäuren codiert werden, können unterschiedliche Degenerationen zum Einsatz kommen (s. Abb. 14). Am häufigsten wird die Kombination NNK (N = G, A, T oder C; K = G oder T) für das zu randomisierende Codon eingesetzt, weil alle AS codiert werden, die Bankgröße gegenüber der Verwendung von NNN nur die Hälfte der Klone beträgt und die Wahrscheinlichkeit für Met und Trp 1/32 gegenüber 1/64 bei NNN ausmacht.

Die minimale Anzahl der Klone, die alle möglichen Einzelmutanten enthalten, ist festgelegt durch die Häufigkeit der am wenigsten repräsentierten Mutanten, d.h. den AS, die nur durch ein Codon codiert werden (N, D, C, E, Q, H, I, K, M, F, W, Y) sowie der Effizienz der eingesetzten Mutagenesemethode. Setzt man ein NNG/T-Codon ein, beträgt die Häufigkeit der am geringsten repräsentierten Mutante (f) ¼ x ¼ x ½ = 1/32. Das bedeutet, dass, eine 100 %ige Mutationseffizienz vorausgesetzt, ca. 100 Klone gescreened werden müssen, um mit 95 %iger Wahrscheinlichkeit alle möglichen Mutanten zu erhalten ([0.95 = 1-(1-f)ⁿ]- n = Anzahl der gescreenten Klone).

Bei der gleichzeitigen Einfügung von zwei NNG/T-Codons ergibt (f): (¼ x ¼ x ½)² = 1/1024 und damit steigt die Zahl der zu screenenden Klone auf 3100; bei drei NNG/T-Codons liegt die Anzahl bei 10⁵ Klonen (Berechnungen aus Hogrefe et al. 2002).

Bei der Inkorporierung von Oligonucleotiden in die codierende Sequenz kann unterschieden werden in Methoden, die den Einbau von Mutationen an verschiedenen/mehreren Stellen der Ziel-DNA erlauben und Techniken, die vor allem für die Inkorporierung eines oder zweier mutagener Oligonukleotide geeignet sind.

Zu der ersten Kategorie gehören z.B. die Methoden ADO (assembly of designed oligonucleotides, Zha et al. 2003) und die multiple-site-directed mutagenesis, beschrieben von Seyfang (2004). Zha et al. setzen überlappende Oligonukleotide ein, die annealen und dann in einer PCR amplifiziert werden, Bei Seyfang et al. hybridisieren Oligonukleotide an eine ssDNA, gefolgt von primer extension und Ligation mit ebenfalls anschließender Amplifikation des mutierten Stranges. Auch Ness et al. (1995) beschreiben ein synthetisches shuffling; diese Autoren rekonstruieren durch überlappende Oligos einen größeren Bereich von DNA.

Hughes et al. (2003) geben mit der sogenannten MAX-Methode die Möglichkeit, eine Mutagenese mit definierten Oligos an mehreren Stellen des Gens vorzunehmen und dabei die Redundanz von Codons zu vermeiden, da jede AS nur durch ein Codon repräsentiert wird. Die Templates der Mutagenese sind randomisierte Oligonukleotide; dieses hat eine Restriktion der Länge des mutagenisierbaren Bereichs zur Folge, da lange Oligonukleotide synthesebedingt Fehler enthalten können. Allerdings könnten hier vielleicht auch zwei (oder mehr) Oligonukleotide annealt werden und in einer primer extension-Reaktion oder overlap-extension PCR das ganze Gen zusammengebaut werden.

Für den Einbau eines oder weniger Oligonukleotide stehen ebenfaüs verschiedene Methoden zur Verfügung, wie z.B. Megaprimer-Techniken (Sarkar und Sommer 1990; Varianten und Weiterentwicklungen von Shepard und Rae, 1999: Tyagi et al. 2004), strand overlap extension (SOE, Higuchi et al. 1988) und QuikChange (Stratagene) basierte Methoden. Hogrefe et al. (2002) benutzen den QuikChange Multi Site-Directed Mutagenesis Kit mit degenerierten Oligonukleotiden als Primern. Zheng et al. (2004) machen sich nur das Prinzip des QuikChange Kits zur nutze; sie setzen aber Primer ein, die nur teilweise überlappen und erreichen so, dass die Bindung der Primer an das Template gegenüber des Self-Pairings bevorzugt ist. Bei der letzteren Methode handelt es sich um eine einfache und anscheinend effiziente Technik.

### b) Robotergestützter Prozess

Zu dem Prinzip des quantitatives Screenings s.o

### (i) Hitpicking mit dem Tecan Freedom 200

Die Hard- und Software sind bei der Firma Tecan kommerziell erhältlich; dabei wurde die Software in Kooperation entwickelt.
In dem Programm Gemini wird das Skript "Colony-Pick" ausgeführt. Dabei wird eingegeben, wie viel % Hits gepickt werden sollen. 70% Ethanol wird im Behälter "Steril 1" für die Sterilisierung der Pipetier- und Pick-Nadeln bereitgestellt. Die gepickten Kolonien werden in Mikrotiterplatten abgesetzt, die das gleiche Selektionsmedium (SC-LWH-Agar für selektionierte Hefekultivierung im 2-Hybrid und N-Hybrid) enthalten wie das, auf dem die zu pickenden Hefen kultiviert wurden.
In der Software Facts wird der Prozess ColonyPicking ausgeführt; hier kann eine Methode ausgewählt werden, wie gescannt werden muss. Für Colony-Pick (Gemini) ist die Methode festgelegt. Und zwar muss für Klone mit dem Redstar- oder dem cobA-Gen als Reportergen die Methode "Redstar-Scanning" mit folgenden Einstellungen ausgeführt werden:
Scan Area: Top 73 mm, Left 2 mm, Bottom 2mm, Right 114 mm
Autofocus: Z-Scan End 1600 µm, Z-Scan Start 1600 µm
Focus Offset: 0 µm, Focal Plane: Plane 1
Laser: 543 nm, Filter: 590 nm, Scan Resolution: 20 µm, Pinhole: Large

Die Omnitray-Platten müssen mit einem Barcode versehen werden.
Nachdem die Kolonien gepickt worden sind und 2 Tage im Brutschrank bei 30 °C kultiviert wurden, werden die Plasmide wieder aus den Hefen isoliert und nach einer Transformation in Bakterien sequenziert.

### (ii) DNA-Isolation aus Hefen mit dem T-Mags von Tecan

Nachdem die Hefen 2 Tage in den Mikrotiterplatten kultiviert wurden, kann nun die DNA-Isolierung aus den Hefen durchgeführt werden. In eine Deepwell-Platte werden in jedes Well 1000 µl Medium (SC-LWH) pipettiert. Nun werden die Kolonien von der Resource-Platte (Platte, auf der die Hefe-Kolonien nach dem Picken wachsen) überimpft in die Deepwell-Platte mit dem jeweiligen Selektionsmedium. Es wird zu den Hefen in der Mikrotiterplatte etwa 200 µl Medium (SC-LWH)/Well pipettiert und durch mehrmaliges Auf- und Ab- pipettieren resuspendiert. Anschließend werden die resuspendierten Hefen überführt in die Deepwell-Platte, in der vorher schon Medium vorgelegt wurde. Nun werden die Hefen etwa 16 Stunden bei 30°C auf einem Mikrotiterplattenschüttler inkubiert.

Am nächsten Tag wird von einigen Wells die optische Dichte bestimmt, indem 100 µl dieser Zellen aus einem Well zu 900 µl Medium gegeben werden. Von dieser 10- fach Verdünnung wird dann die optische Dichte bestimmt. Hiermit bestimmt man dann den Mittelwert von allen Wells. Die Deepwell-Platte mit den Zellen wird dann bei 2500 rpm, 5 Minuten im Ausschwingrotor zentrifugiert (Sorvall Zentrifuge). Als Gegengewicht wird eine andere Deepwell-platte mit der gleichen Volumen mit H₂O befüllt. Nach dem Zentrifugieren wird durch Dekantieren der Deepwellplatte der Überstand entfernt. In jedes Well werden 300 µl Y1-Puffer pipettiert. Zu jedem Well werden außerdem 1-2 Units Lyticase/OD₆₀₀ (der Hefen in den Wells) pipettiert. Puffer und Lyticase werden gut mit den Zellen vermischt. Die Deepwell-Platte wird 1,5 Stunden bei 30°C inkubiert (Nicht schütteln). Die Deepwell-Platte wird bei 2500 rpm, 5 Minuten im Ausschwingrotor zentrifugiert (Sorvall Zentrifuge). Nach dem Zentrifugieren wird durch Dekantieren der Deepwellplatte der Überstand entfernt. Die Zellen in der Deepwell-Platte werden in 250 µl ddH₂O aufgenommen. Die Zellen müssen gut resuspendiert werden. Die Deepwell-Platte ist damit fertig für die DNA-Isolierung.

Mit der Software Gemini des Tecan-Roboters wird eine Methode zur Isolation der DNA ausgeführt, die von der Firma AGOWA (Berlin) zusammen mit Tecan entwickelt worden ist. Diese Isolation findet in dem Gerät T-Mags auf der Roboterplattform statt.

### (iii) Transformation von Bakterien

### Herstellung kompetenter Bakterien zur Transformation In PCR-Platten

5 ml SOB-Medium werden mit einer Einzelkolonie *E.coli*-DH5□-Bakterien angeimpft. Die Zellen werden über Nacht bei 37 °C im Schüttler (210-225 rpm) inkubiert. 50-100 µl dieser Kultur werden in 100 ml SOB-Medium überführt und bei 37 °C im Schüttler (180 rpm) inkubiert. Bei OD₆₀₀ = 0,1 bis 0,5 (nach ca. 2-3 Stunden) werden die Bakterien geerntet und für 20 min auf Eis gestellt. Ab hier erfolgen alle weiteren Schritte bei einer Temperatur von 4 °C, Die Bakterienkultur wird im 50 ml Falcongefäß (Spitzboden) bei 4 °C und 1200 g zentrifugiert. Das Pellet wird in 10 ml eiskalter 50 mM CaCl2-Lösung durch Auf- und Abpipettieren resuspendiert und anschließend für mindestens 30 min auf Eis inkubiert. Nun werden die Zellen bei 4 °C und 1200 g 5 min zentrifugiert. Die Zellen werden in 1 ml/0,1 OD₆₀₀ eiskalter 50 mM CaCl2-Lösung mit 15 % Glycerin durch Auf- und Abpipettieren resuspendiert. Bei einer OD₆₀₀ = 0,1 werden die Zellen also in 1ml, bei einer OD₆₀₀ = 0,3 in 3 ml CaCl2 aufgenommen. 10 µl-Aliquots pro Well werden in eine auf Eis vorgekühlte PCR-Platte gefüllt und bei -80 °C eingefroren und gelagert.

### Transformation der Bakterien mit der DNA aus Hefen

Das Auftauen der kompetenten Bakterien in den PCR-Platten erfolgt in einem PCR-Block aus Metall, der auf Eis steht. Zu jedem Well werden 10 µl isolierte DNA hinzupipettiert. Bakterien und DNA werden vorsichtig gemischt (nicht Auf- und Abpipettieren!). Die Zellen werden nun mindestens 30 min auf Eis inkubiert. Anschließend wird ein Hitzeschock von 30 s auf einem Heizblock bei 42 °C ausgeführt. Nach dem Hitzeschock kommen die Zellen erneut für 2 min auf Eis. In einer Deepwell-Platte werden 100 µl SOC Medium vorgelegt. Zu den Bakterien werden auch 100 µl SOC pro Well hinzupipettiert. Die Bakterien werden von der PCR-Platte in die Deepwell-Platte überführt und für 1 Stunde bei 37 °C in dem Mikrotiterplattenschüttler bei 210 bis 225 rpm inkubiert. Nach 1 Stunde Inkubation wird 1 ml LB-Medium mit entsprechendem Antibiotikum zu den Zellen hinzupipettiert und für mindestens 20 Stunden bei 37 °C in dem Mikrotiterplattenschüttler bei 210 bis 225 rpm inkubiert. Nach dieser Inkubation werden 5-10 µl der Zellen auf eine 96-Well Platte mit LB-Agar + Antibiotikum überführt und diese Platte wird 16 Stunden bei 37°C im Brutschrank inkubiert. Diese Platte kann zur Sequenzierung der einzelnen Kolonien zu AGOWA geschickt werden.

### c) Ausführungsbeispiel

Mutagenese auf RafRBD A85

### (i) Konstruktion der notwendigen Vektoren

Nach der Transformation der mutagenisierten Bank befinden sich zwei Plasmide in der Hefe, die beide ein Ampicillin-Resistenzgen tragen. Zum Einen pPC97 mit dem ras-Gen (im erfindungsgemäßen Verfahren befindet sich auf diesem Plasmid noch zusätzlich der Kompetitor) und zum Anderen pPC86, von dem das mutierte RafRBD-Gen codiert wird. Nach der Transformation der aus diesen Hefen isolierten DNA in kompetente Bakterien soll nur noch das Plasmid, auf dem das mutierte RafRBD-Gen vorhanden ist, in den Bakterien vorliegen. Zu diesem Zweck muss einer der Vektoren mit einer anderen Antibiotikumresistenz ausgestatten werden. Im vorliegenden Fall wurde pPC86 mit einer Kanamycinresistenz versehen. Hierzu wurde vor und nach dem TEM-Resistenzgen mittels QuikChange Mutation nach Angaben des Herstellers jeweils eine Pmel-site generiert, das Gen danach ausgeschnitten und durch das Kanamycin-Resistenzgen ersetzt. Die mit der DNA aus Hefe transformierten Bakterien wachsen nun in Medium mit Kanamycin und können so von den Bakterien, die das Fusions-Köderprotein-Plasmid pPC97 enthaiten, separiert werden.

### Verwendete Primer:

Multi-QC-Pme-vor-TEM
   TGAATACTCATACTCTTCCTGTTTAAACATTATTGAAGCATTTATCAGGG
Multi-QC-Pme-nach-TEM:
   TTAAATCAATCTAAAGTATATATGTTTAAACTTGGTCTGACAGTTACCAATG (Pmel)
Pme-Kan-for:
   AAAAAACCGTTTAAACAGGAAGAGTATGATTGAACAAGATGGATTGC (Pmel) Pme-Kan-rev:
   AAAAAACCGTTTAAACTTGGTCTGACAGTCAGAAGAACTCGTCAAGAAGG (Pmel)

### (ii) Durchführung der Zufallsmutagenese

Die Zufallsmutagenese wird nach der Methode von Zheng et al. (2004) durchgeführt (s. Abschnitt b). Hierzu wurden folgende zwei Primer entworfen, die teilweise überlappen und die Aminosäure A85 von RafRBD randomisieren:
Z-RBD-A85-forl: CTGCCTTATGAAANNKCTCAAGGTGAGGGGCCTGCAACCAG
Z-RBD-A85-rev CCCTCACCTTGAGMNNTTTCATAAGGCAGTCATGCAAGCTC

Diese Primer werden zu einer PCR mit dem Expand Kit (Roche) eingesetzt. Hierfür werden 50 ng Template-DNA (pPC86-RafRBD mit Kanamycin-Resistenzgen) verwendet und die PCR nach Angaben des Herstellers mit 0,8 pmol/µl jedes Primers durchgeführt. Die PCR-Reaktion wird dann mit dem PCR Purification Kit (Qiagen) aufgereinigt und 5 von 50 µl werden auf ein Agarosegel aufgetragen. Der restliche Ansatz wird mit 10 U Dpnl (NEB, in Puffer 4) 3 Stunden bei 37 °C restringiert, um die methylierte, da aus *E.coli* isolierte, Template-DNA zu entfernen. Zheng et al. führten hier den Verdau nur 1 Stunde durch, was aber in einem hohen Hintergrund an Wildtyp-Klonen in der Bank resultierte.

Transformiert werden dann 2,5 µl des Dpnl-Cuts in 75 µl kompetente XL10 Gold-Zellen (Stratagene) nach Angaben des Herstellers; nach Zugabe von 750 µl NZY nach dem Hitzeschock erfolgt eine einstündige Regenerationsphase der Bakterien. 20 µl des Transformationsansatzes werden zur Bestimmung der Transformationseffizienz ausplattiert (ca. 1/20 des Gesamtansatzes) und der Rest wird in LB-Medium mit Kanamycin über Nacht bei 37 °C und 225 rpm auf dem Schüttler inkubiert. Am nächsten Morgen wird die DNA isoliert. Die Bestimmung der Anzahl an voraussichtlich unabhängigen Kolonien erfolgt durch Zählen der ausplattierten Kolonien und Hochrechnen auf die Gesamtzahl (Faktor 20). Diese Zahl wird dann durch 4 geteilt, weil angenommen wird, dass zwei Teilungen der Bakterien in der einstündigen Regenerationszeit stattfinden. Dieser Wert sollte deutlich über 100 liegen, damit eine repräsentative Bank angenommen werden kann (s. Berechnungen im Abschnitt Zufallsmutagenese). Im vorliegenden Fall wurden 152 Kolonien nach der Transformation gezählt, was eine Zahl von ca 760 unabhängigen Kolonien im Ansatz bedeutet.

Die Bank wird durch Sequenzierung von Einzelkolonien und der Bank-DNA charakterisiert. Diese DNA wird dann in die Hefe transformiert, die RedStar als Reportergen im Genom enthält. Dabei wird einmal pPC97-ras (für das Two-Hybrid) und einmal pPC97-ras mit Kompetitor TEF-Promotor/RafRBD (für das n-Hybrid-System, erfindungsgemäße Verfahren) als zweites Plasmid eingesetzt.

### (iii) robotergestützter Prozess

Es wird ein quantitatives Screening auf Hefekolonien durchgeführt, die auf Medium mit 50 mM 3-AT gewachsen waren. Hitpicking, DNA-Isolation und Bakterientransformation werden wie oben beschrieben durchgeführt.

### (iv) Sequenzierung und Auswertung der Hits

Ergebnis der Sequenzierung der DNA aus den Bakterienkolonien

**Tab 8: Ergebnis des Hitpickings mit RedStar als Reportergen und bei Expression des Kompetitors RafRBD-wt unter Regulation des TEF Promotors**

| | n-Hybrid (18 Kolonien) | |
|---|---|---|
| codierte AS | Anzahl | % |
| K (Lys) | 12 | 66,7 |
| R (Arg) | 3 | 16,7 |
| P (Pro) | 2 | 11,1 |
| S (Ser) | 1 | 5,6 |

Ganz deutlich wird, dass mit Hilfe des erfindungsgemäßen Verfahrens die Diskriminierung zwischen der in der Literatur beschriebenen verbesserten Mutante A85K (Fridman et al., 2000) und dem Wildtyp sehr gut möglich ist: Es ist kein einziges Mal Wildtyp gepickt worden. Dagegen ist in dem bekannten Two-Hybrid-System, in dem die gleiche DNA mit der randomisierten Position eingesetzt wurde, in 21,2 % der Fälle der Wildtyp gepickt worden. Die verbesserte Mutante mit K (Lysin) wurde lediglich zu 27,3 % gefunden. Dieses Ergebnis bestätigt nochmals die bereits beschriebenen Schwierigkeiten (s. Tab. 1) bei der Diskriminierung von A85K gegenüber dem Wildtyp im Two-Hybrid-System nach dem Stand der Technik. Diese Daten zeigen somit die Überlegenheit des erfindungsgemäßen Verfahrens.

### Literatur:

Arntz, C., Meinders, D., Block, C., Mittmann, K, (2004) Phycocyanin exprimierende Eukaryontenzelle. EPA 04 001 504.2
Aronheim. A., Zandi, E., Elledge, S.J. und Karin, M. (1997) Isolation of an AP-1 Repressor by a novel Method for detecting Protein-Protein Interactions. Mol. Cell. Biol. 17, 3094-3102
Bitter GA and Egan KM (1984) Expression of heterologous genes in Saccharomyces cerevisiae from vectors utilizing the glyceraldehydes-3-phosphate dehydrogenase gene promoter. Gene 32:263-274
Boeke JD, LaCroute F, Fink GR. (1984) A positive selection for mutants lacking orotidine-5'-phosphate decarboxylase activity in yeast: 5-fluoro-orotic acid resistance. Mol. Gen. Genet. 197(2):345-6
Botstein D, Falco S et al. (1979) Sterile host yeasts (SHY): A eukaryotic system of biological containment for recombinant DNA experiments. Gene 8:17-24
Bulter T und Alcalde M (2003) Preparing Libraries in Saccharomyces cerevisiae. Methods in Molecular Biology 231: 17-22,
Directed Evolution Library Creation: Methods and Protocols, Humana Press Chevray PM, Nathans D. (1992)
Protein interaction cloning in yeast: identification of mammalian proteins that react with the leucine zipper of Jun. PNAS 89(13): 5789-5793
Cottrelle P, Thiele D, Price V, Memet S, Micouin J-Y, Marck C, Buhler J-M, Sentenac A, Fromageot P (1985) Cloning, Nucleotide Sequence, and Expression of One of Two Genes Coding for Yeast Elonation Factor 1a. JBC 260:3090-3096
de Felipe, K.S., Carter, B.T., Althoff, E.A. und Comish, V.W. (2004) Correlation between Ligand-Receptor Affinity and the Transkription Readout in a Yeast Three-Hybrid System. Biochemistry 43, 10535-10363
Estojak, J., Brent, R. und Golemis, E. (1995) Mol. Cell. Biol. 15, 5820-5829 Fields, S. Song, O. (1989) A novel genetic system to detect protein-protein interactions. Nature 340, 245-246
Fridman, M., Maruta, H., Gonez, J., Walker, F., Treutlein, H., Zeng, J. und Burgess, A. (2000) Point Mutants of c-raf-1 RBD with elevated Binding to v-Ha-Ras. J. Biol. Chem. 275, 30363-30371
Fujii R, Kitaoka M, Hayashi K (2004) One-step random mutagenesis by errorprone rolling circle amplification, Nucleic Acids Research 32(19) e145
Fuller RS, Brake A, Thorner J (1989) Yeast prohormone processing enzyme (KEX2 gene product) is a Ca2+-dependent serine protease. PNAS 86:1434-1438
Guarente L, Ptashne M (1981) Fusion of Escherichia coli lacZ to the cytochrome c gene of Saccharomyces cerevisiae. PNAS 78:2199-2203
Higuchi T, Krummel B, Saiki RK (1988) A general method for in vitro preparation and specific mutagenesis of DNA fragments: study of protein and DNA interactions. Nucleic Acids Research 16:7351-7367
Hogrefe HH, Cline J, Youngblood GL. Allen RM (2002) Creating Randomized Amino Acid Libraries with the QuikChange Multi Site-Directed Mutagenesis Kit. BioTechniques 33(5):1158-1165
Hua S-B, Qiu M, Chan E, Zhu L, Luo Y (1997) Minimum Length of Sequence Homology Required for in Vivo Cloning by Homologous Recombination in Yeast. Plasmid 38: 91-96
Hughes MD, Nagel DA, Santos AF, Sutherland AJ, Hine AV (2003) Removing the Redundancy From Randomised Gene Libraries. J. Mol. Biol. 331:973-979
Jaitner, B.K., Becker, J., Linnemann, T., Herrmann, C., Wittinghofer, A. und Block C. (1997) Discrimination of Amino Acids mediating Ras binding from noninteracting Residues affecting Raf Activation by Double Mutant Analysis. J. Biol. Chem. 272, 29927-29933
Knop M, Barr F, Riedel CG, Heckel T, Reichel C (2002) Improbed Version of the Red Fluorescent Protein (drFP583/DsRed/RFP). BioTechniques 33:592-602
Knop, M., Barr, F., Riedel, C.G., Heckel, T. and Reichel, C. (2002) Improved version of the red fluorescent protein (drFP583/DsRed/RFP). Biotechniques 33, 592-602.
Leuther KK, Johnston SA (1992) Nondissociation of GAL4 and GAL80 in Vivo ter Galactose Induction. Science 256:1333-1335
Nacken V, Achstetter T, Degryse E (1996) Probing the limits of expression levels by varying promoter strength and plasmid copy numer in Saccharomyces cerevisiae. Gene 175;2563-260
Ness JE, Kim S, Gottman A et al. (1995) Synthetic shuffling expands functional protein diversity by allowing amino acids to recombine independently. Nature Biotechnology 20:1251-1255
Neylon C (2004) Chemical and biochemical strategies fort the randomization of protein encoding DNA sequences: library construction methods for directed evolution. Nucleic Acids Research 32(4):1448-1459
Osborne, M.A., Dalton, S. und Kochan, J.P. (1995) The Yeast Tribrid System-Genetic Detection of trans-phosphorylated ITAM-SH2-Interactions. Bio/Technology 13, 1474-1478
Roessner C.A. (2002) Use of cobA and cysGA as red fluorescent indicators. Methods Mol. Biol. 183, 19-30
Roessner CA and Scott AI (1995) Fluorescence-based method for selection of recombinant plasmids. BioTechniques 19:760-764
Sandrock, B. und Egly, J.M. (2001) A Yeast Four-Hybrid System identifies CDKaktivating Kinase as a Regulator of the XPD Helicase, a Subunit of Transcription Factor IIH. J. Biol. Chem. 276, 35328-35333
Sarkar G and Sommer, SS (1990) The "megaprimer" method of site-directed mutagenesis. BioTechniques 8:404-407
Selzer, T., Albeck, S. und Schreiber, G. (2000) Rational design of faster associating and tighter binding complexes. Nature Struct. Biol. 7, 537-541
Serebrüskü, 1., Khazak, V. und Golemis, E.A. (1999) A Two-Hybrid dual Bait System to Discriminate Specificity of Protein Interactions. J. Biol. Chem. 274, 17080-17087
Seyfang A and Jin JH (2004) Multiple site-directed mutagenesis of more than 10 sites simultaneously and in a single round. Analytical Biochemistry 324:285-291
Shepard AR and Rae JL (1999) Simple Version of "Megaprimer" PCR for Site-Directed Mutagenesis. BioTechniques 26:870-873
Tirode, F., Malaguti, C., Romero, F., Attar, F., Camonis, J. und Egly, J.M. (1997) A conditionally expressed third Partner stabilizes or prevents the Formation of a Transcriptional Activator in a Three-Hybrid System. J. Biol, Chem. 272, 22995-22999
Treco DA Basic Techniques of Yeast Genetics (1989) In: Current Protocols in Molecular Biology, Wiley, Supplement 5, Unit 13.1, p 13.1.5
Tyagi R, Lai R, Duggleby RG (2004) A new approach to "megaprimer" polymerase chain reaction mutagenesis without an intermediate gel purification step. BMC Biotechnology 4:2-7
Vidal, M., Brachmann, R.K., Fattaey, A., Harlow, E. und Boeke, J. (1996) Reverse Two-Hybrid and One-Hybrid Systems to detect dissociation of protein-protein and DNA-protein interactions. Proc. Natl. Acad. Sci. USA 93, 10315-10320
West RW, Yocum RR. Ptashne M. (1984) Saccharomyces cerevisiae GAL1-GAL10 Divergent Promoter Region: Location and Function of the Upstream Activating Sequence UASG. Mol Cell Biol Nov.1984:2467-2478
Wildt S and Deuschle U (1999) cobA, a red fluorescent transcriptional reporter for Escherichia coli, yeast, and mammalian cells. Nat Biotech 17:1175-1178
Wong TS, Tee KL, Hauer B, Schwaneberg U (2004) Sequence saturation mutagenesis (SeSaM): a novel method for directed evolution. Nucleic Acids Research 32(3): e26
Yocum RR, Hanley S, West R, Ptashne M (1984) Use of lacZ Fusions to Delimit Regulatory Elements of the Inducible Divergent GAL1-GAL10 Promoter in Saccharomyces cerevisiae. Mol Cell Biol Oct. 1984:1985-1998
Zaccolo M, Williams DM, Brown DM, Gherardi E (1996) mAn approach to random mutagenesis of DNA using mixtures oftriphosphate derivatives of nucleoside analogues. J. Mol. Biol. 255: 589-603
Zha D, Eipper A, Reetz MT (2003) Assembly of Designed Oligonucleatides as an Efficient Method for Gene Recombination: A New Tool in Directed Evolution. ChemBioChem 4: 34-39
Zhang, J. und Lautar. S. (1996) A Yeast Three-Hybrid Method to clone Temary Protein Complex Components. Anal. Biochem. 242, 68-72
Zheng L, Baumann U, Reymond J-L (2004) An efficient one-step site-directed and site-saturation mutagenensis protocol. Nucleic Acids Research 32(14): e115

## Patentansprüche

1. Verfahren zur Identifikation hochaffiner Liganden umfassend folgende Schritte:
a) Erzeugen einer Bank für ein mutagenisiertes erstes Hybridprotein umfassend eine Vielzahl von Mutanten;
b) Expression des ersten Hybridproteins in einem Wirt mit einem zweiten Hybridprotein, wobei eines der Hybridproteine die DNA-Bindedomäne eines Transkriptionsfaktors und ein Köderprotein und das andere der Hybridproteine die Aktivierungsdomäne für einen Transkriptionsfaktor und ein Beuteprotein umfaßt;
c) Ermöglichen der Bindungsreaktion zwischen dem ersten und dem zweiten Hybridprotein zu einem Komplex mit funktionalem Transkriptionsfaktor in der Wirtszelle, wobei die Lage des Gleichgewichts durch den Einsatz eines in der Wirtszelle exprimierten Kompetitors für mindestens ein Hybridprotein zu Gunsten der Ausgangsprodukte verschoben wird;
d) Nachweis der Bindungsreaktion durch den Nachweis eines über den funktionalen Transkriptionsfaktor exprimierten Reportergens;
e) ggf. Wiederholen eines Schrittes oder mehrerer Schritte von a) bis d);
f) Auswahl einer Mutante.

2. Verfahren nach Anspruch 1, **dadurch gekennzeichnet, daß** die Konzentration des Kompetitors variiert wird.

3. Verfahren nach Anspruch 1 oder 2, **dadurch gekennzeichnet, daß** die Expression des Kompetitors durch die Wahl eines geeigneten Promotors reguliert wird.

4. Verfahren nach einem der Ansprüche 1 bis 3, **dadurch gekennzeichnet, daß** die Wirtszelle auf einem Selektionsmedium kultiviert wird.

5. Verfahren nach einem der vorherigen Ansprüche, **dadurch gekennzeichnet, daß** die Bindungsreaktionen mindestens zweier verschiedener Hybridproteine miteinander verglichen werden.

6. Verfahren nach Anspruch 5, **dadurch gekennzeichnet, daß** die Bindungsreaktion eines mutagenisierten Hybridproteins mit seinem Wildtyp verglichen wird.

7. Wirtszelle codierend ein erstes Hybridprotein und ein zweites Hybridprotein, wobei die Hybridproteine miteinander einen funktionellen Ligandenkomplex bilden können, sowie ein Protein, das zu einem der Hybridproteine einen Kompetitor darstellt.

8. System aus einer Wirtszelle codierend ein erstes Hybridprotein und ein zweites Hybridprotein, wobei die Hybridproteine miteinander einen funktionellen Ligandenkomplex bilden können, und einem Protein, das zu einem der Hybridproteine einen Kompetitor darstellt.

9. Plasmid codierend ein mit einem zweiten Hybridprotein einen Ligandenkomplex bildendes erstes Hybridprotein und ein weiteres Protein, das zu dem ersten oder zweiten Hybridprotein einen Kompetitor darstellt.

10. Verwendung der Wirtszelle nach Anspruch 7 oder des Systems nach Anspruch 8 oder des Plasmids nach Anspruch9 zur Bestimmung von Bindungsaffinitäten.

11. Verfahren zur Identifikation hochaffiner Liganden umfassend folgende Schritte:
a) Erzeugen einer Bank für ein mutagenisiertes erstes Hybridprotein umfassend eine Vielzahl von Mutanten;
b) Expression des ersten Hybridproteins in einem Wirt mit einem zweiten Hybridprotein, wobei eines der Hybridproteine die DNA-Bindedomäne eines Transkriptionsfaktors und ein Köderprotein und das andere der Hybridproteine die Aktivierungsdomäne für einen Transkriptionsfaktor und ein Beuteprotein umfaßt;
c) Ermöglichen der Bindungsreaktion zwischen dem ersten und dem zweiten Hybridprotein zu einem Komplex mit funktionalem Transkriptionsfaktor in der Wirtszelle, wobei die Lage des Gleichgewichts durch den Einsatz eines Kompetitors für mindestens ein Hybridprotein zu Gunsten der Ausgangsprodukte verschoben wird,
d) Nachweis der Bindungsreaktion durch den Nachweis eines über den funktionalen Transkriptionsfaktor exprimierten Reportergens;
e) Wiederholen der Schritte von a) bis d), wobei der Kompetitor eine gegenüber dem Kompetitor nach Schritt c) verbesserte Affinität zu den Hybridproteinen aufweist und
f) Auswahl einer affinen Mutante.

## Claims

1. Method for identifying high-affinity ligands, comprising the following steps:
a) producing a library for a mutagenised first hybrid protein comprising a plurality of mutants;
b) expressing the first hybrid protein in a host with a second hybrid protein, one of the hybrid proteins comprising both the DNA binding domain of a transcription factor and a bait protein, and the other of the hybrid proteins comprising both the activation domain for a transcription factor and a prey protein;
c) enabling the binding reaction between the first and second hybrid proteins to form a complex with a functional transcription factor in the host cell, the position of the equilibrium being shifted in favour of the starting products by using a competitor, expressed in the host cell, for at least one hybrid protein;
d) detecting the binding reaction by detecting a reporter gene expressed via the functional transcription factor;
e) optionally repeating one or more of steps a) to d);
f) selecting a mutant.

2. Method according to Claim 1, **characterized in that** the concentration of the competitor is varied.

3. Method according to Claim 1 or 2, **characterized in that** the expression of the competitor is regulated by the selection of a suitable promoter.

4. Method according to any one of Claims 1 to 3, **characterized in that** the host cell is cultivated on a selection medium.

5. Method according to any one of the preceding claims, **characterized in that** the binding reactions of at least two different hybrid proteins are compared with one another.

6. Method according to Claim 5, **characterized in that** the binding reaction of a mutagenised hybrid protein is compared with its wild type.

7. Host cell coding for a first hybrid protein and a second hybrid protein, the hybrid proteins together being able to form a functional ligand complex, and a protein which represents a competitor to one of the hybrid proteins.

8. System comprising a host cell coding for a first hybrid protein and a second hybrid protein, the hybrid proteins together being able to form a functional ligand complex, and a protein which represents a competitor to one of the hybrid proteins.

9. Plasmid coding for a first hybrid protein, which forms a ligand complex with a second hybrid protein, and a further protein which represents a competitor to the first or second hybrid protein.

10. Use of the host cell according to Claim 7 or the system according to Claim 8 or the plasmid according to Claim 9 to determine binding affinities.

11. Method for identifying high-affinity ligands, comprising the following steps:
a) producing a library for a mutagenised first hybrid protein comprising a plurality of mutants;
b) expressing the first hybrid protein in a host with a second hybrid protein, one of the hybrid proteins comprising both the DNA binding domain of a transcription factor and a bait protein, and the other of the hybrid proteins comprising both the activation domain for a transcription factor and a prey protein;
c) enabling the binding reaction between the first and second hybrid proteins to form a complex with a functional transcription factor in the host cell, the position of the equilibrium being shifted in favour of the starting products by using a competitor for at least one hybrid protein;
d) detecting the binding reaction by detecting a reporter gene expressed via the functional transcription factor;
e) repeating steps a) to d), the competitor having an improved affinity for the hybrid proteins compared with the competitor according to step c) and
f) selecting an affine mutant.

## Revendications

1. Procédé d'identification de ligands à haute affinité, comprenant les étapes suivantes:
a) Production d'une banque d'une première protéine hybride mutée comprenant une pluralité de mutants;
b) Expression de la première protéine hybride dans un hôte avec une deuxième protéine hybride, une des protéines hybrides comprenant le domaine de liaison à l'ADN d'un facteur de transcription et une protéine appât et l'autre des protéines hybrides comprenant le domaine d'activation d'un facteur de transcription et une protéine proie;
c) Rendre possible la réaction de liaison entre la première et la deuxième protéine hybride en un complexe avec un facteur de transcription fonctionnel dans la cellule hôte, la position d'équilibre étant déplacée en faveur des produits de départ, par mise en oeuvre d'un compétiteur exprimé dans la cellule hôte pour au moins une protéine hybride;
d) Mise en évidence de la réaction de liaison par détection d'un gène reporter exprimé par le facteur de transcription fonctionnel;
e) Optionellement, répétition d'une ou plusieurs étapes a) à d);
f) Choix d'un mutant.

2. Procédé selon la revendication 1, **caractérisé en ce que** la concentration du compétiteur est variée.

3. Procédé selon la revendication 1 ou 2, **caractérisé en ce que** l'expression du compétiteur est régulée par le choix d'un promoteur approprié.

4. Procédé selon l'une des revendications 1 à 3, **caractérisé en ce que** la cellule hôte est cultivée sur un milieu de sélection.

5. Procédé selon l'une des revendications précédentes, **caractérisé en ce que** les réactions de liaison d'au moins deux protéines hybrides différentes sont comparées l'une à l'autre.

6. Procédé selon la revendication 5, **caractérisé en ce que** la réaction de liaison d'une protéine hybride mutée est comparée à son type sauvage.

7. Cellule hôte codant une première protéine hybride et une deuxième protéine hybride, les protéines hybrides pouvant former l'une avec l'autre, un complexe ligand fonctionnel, ainsi qu'une protéine, qui représente un compétiteur pour une des protéines hybrides.

8. Système consistant en une cellule hôte codant une première protéine hybride et une deuxième protéine hybride, les protéines hybrides pouvant former l'une avec l'autre, un complexe ligand fonctionnel, ainsi qu'une protéine, qui représente un compétiteur pour une des protéines hybrides.

9. Plasmide codant une première protéine hybride, formant un complexe ligand avec une deuxième protéine hybride, et une autre protéine, qui représente un compétiteur pour la première ou la deuxième protéine hybride.

10. Utilisation de la cellule hôte selon la revendication 7 ou du système selon la revendication 8 ou du plasmide selon la revendication 9, pour déterminer des affinités de liaison.

11. Procédé d'identification de ligands à haute affinité, comprenant les étapes suivantes:
a) Production d'une banque d'une première protéine hybride mutée comprenant une pluralité de mutants;
b) Expression de la première protéine hybride dans un hôte avec une deuxième protéine hybride, une des protéines hybrides comprenant le domaine de liaison à l'ADN d'un facteur de transcription et une protéine appât et l'autre des protéines hybrides comprenant le domaine d'activation d'un facteur de transcription et une protéine proie;
c) Autorisation de (rendre possible) la réaction de liaison entre la première et la deuxième protéine hybride en un complexe avec un facteur de transcription fonctionnel dans la cellule hôte, la position d'équilibre étant déplacée en faveur des produits de départ, par mise en oeuvre d'un compétiteur pour au moins une protéine hybride;
d) Mise en évidence de la réaction de liaison par détection d'un gène reporter exprimé par le facteur de transcription fonctionnel;
e) Répétition des étapes a) à d), le compétiteur présentant une affinité améliorée pour les protéines hybrides, par rapport au compétiteur de l'étape c);
f) Choix d'un mutant avec affinité.
